(19) 

**Europäisches Patentamt**
**European Patent Office**
**Office européen des brevets**

(11) **EP 1 925 323 A1**

(12) **EUROPEAN PATENT APPLICATION**

(43) Date of publication:
  **28.05.2008 Bulletin 2008/22**

(51) Int Cl.:
  **A61L 15/28** (2006.01)

(21) Application number: **07253765.7**

(22) Date of filing: **24.09.2007**

(84) Designated Contracting States:
  **AT BE BG CH CY CZ DE DK EE ES FI FR GB GR HU IE IS IT LI LT LU LV MC MT NL PL PT RO SE SI SK TR**
  Designated Extension States:
  **AL BA HR MK RS**

(30) Priority: **02.10.2006 US 537849**
      **02.10.2006 US 537989**
      **02.10.2006 US 610353**

(71) Applicant: **WEYERHAEUSER COMPANY**
  **Federal Way, WA 98063-9777 (US)**

(72) Inventors:
  • **Weerawarna, S. Ananda**
    **Seattle, WA 98125 (US)**
  • **Luo, Mengkui**
    **Auburn, WA 98001 (US)**
  • **Bing, Su**
    **Federal Way, WA 98032 (US)**
  • **Michalek, Alena**
    **Auburn, WA 98001 (US)**

(74) Representative: **Hayes, Adrian Chetwynd et al**
  **Boult Wade Tennant,**
  **Verulam Gardens**
  **70 Gray's Inn Road**
  **London WC1X 8BT (GB)**

(54) **Mixed polymer superabsorbent fibers and method for their preparation**

(57)   A mixed polymer composite fiber including a carboxyalkyl cellulose and a galactomannan polymer or glucomannan polymer, and methods of making the fiber by blending the polymers in water, crosslinking them and forming fibers..

EP 1 925 323 A1

**Description**

BACKGROUND OF THE INVENTION

Personal care absorbent products, such as infant diapers, adult incontinent pads, and feminine care products, typically contain an absorbent core that includes superabsorbent polymer particles distributed within a fibrous matrix. Superabsorbents are water-swellable, generally water-insoluble absorbent materials having a high absorbent capacity for body fluids. Superabsorbent polymers (SAPs) in common use are mostly derived from acrylic acid, which is itself derived from petroleum oil, a non-renewable raw material. Acrylic acid polymers and SAPs are generally recognized as not being biodegradable. Despite their wide use, some segments of the absorbent products market are concerned about the use of non-renewable petroleum oil derived materials and their non-biodegradable nature. Acrylic acid based polymers also comprise a meaningful portion of the cost structure of diapers and incontinent pads. Users of SAP are interested in lower cost SAPs. The high cost derives in part from the cost structure for the manufacture of acrylic acid which, in turn, depends upon the fluctuating price of petroleum oil. Also, when diapers are discarded after use they normally contain considerably less than their maximum or theoretical content of body fluids. In other words, in terms of their fluid holding capacity, they are "over-designed". This "over-design" constitutes an inefficiency in the use of SAP. The inefficiency results in part from the fact that SAPs are designed to have high gel strength (as demonstrated by high absorbency under load or AUL). The high gel strength (upon swelling) of currently used SAP particles helps them to retain a lot of void space between particles, which is helpful for rapid fluid uptake. However, this high "void volume" simultaneously results in there being a lot of interstitial (between particle) liquid in the product in the saturated state. When there is a lot of interstitial liquid the "rewet" value or "wet feeling" of an absorbent product is compromised.

In personal care absorbent products, U.S. southern pine fluff pulp is commonly used in conjunction with the SAP. This fluff is recognized worldwide as the preferred fiber for absorbent products. The preference is based on the fluff pulp's advantageous high fiber length (about 2.8 mm) and its relative ease of processing from a wetland pulp sheet to an airlaid web. Fluff pulp is also made from renewable and biodegradable cellulose pulp fibers. Compared to SAP, these fibers are inexpensive on a per mass basis, but tend to be more expensive on a per unit of liquid held basis. These fluff pulp fibers mostly absorb within the interstices between fibers. For this reason, a fibrous matrix readily releases acquired liquid on application of pressure. The tendency to release acquired liquid can result in significant skin wetness during use of an absorbent product that includes a core formed exclusively from cellulosic fibers. Such products also tend to leak acquired liquid because liquid is not effectively retained in such a fibrous absorbent core.

Superabsorbent produced in fiber form has a distinct advantage over particle forms in some applications. Such superabsorbent fiber can be made into a pad form without added non superabsorbent fiber. Such pads will also be less bulky due to elimination or reduction of the non superabsorbent fiber used. Liquid acquisition will be more uniform compared to a fiber pad with shifting superabsorbent particles.

A need therefore exists for a fibrous superabsorbent material that is simultaneously made from a biodegradable renewable resource like cellulose that is inexpensive. In this way, the superabsorbent material can be used in absorbent product designs that are efficient. These and other objectives are accomplished by the invention set forth below.

SUMMARY OF THE INVENTION

The present invention provides a mixed polymer composite fiber. The mixed polymer composite fiber includes a carboxyalkyl cellulose and a galactomannan polymer or a glucomannan polymer. The fiber includes a plurality of non-permanent intra-fiber metal crosslinks.

The present invention provides a method for making the mixed polymer composite fibers.

In the method, a carboxyalkyl cellulose and a galactomannan polymer or glucomannan polymer are blended in water to provide an aqueous solution; the aqueous solution treated with a first crosslinking agent to provide a gel.

In one embodiment the gel is mixed with a water-miscible solvent to provide fibers; and the fibers treated with a second crosslinking agent to provide crosslinked mixed polymer composite fibers.

In another embodiment the gel is then spun into gel fibers using centrifugal spinning. In another embodiment the gel is extruded into gel fibers using meltblowing. In another embodiment the gel is formed into gel fibers using wet spinning.

In an embodiment the spun or extruded gel fibers are precipitated into solid fibers by being passed into a solvent bath to provide mixed polymer composite fibers. In another embodiment the spun or extruded gel fibers are precipitated into solid fibers by being sprayed with a solvent to provide mixed polymer composite fibers. The solvent bath or spray uses a water miscible solvent.

In another embodiment the bath or spray may contain a second crosslinking agent to provide further crosslinking of the fibers.

The mixed polymer composite fibers may then be dried.

The spun or extrusion embodiments allows fibers of a specific and predetermined diameter and cross-section to be formed. The fibers may have diameter of 50 μm to 1000 μm. In some instances the diameter of the fibers may vary along the fiber length.

DESCRIPTION OF THE DRAWINGS

The foregoing aspects and many of the attendant advantages of this invention will become more readily appreciated as the same become better understood by reference to the following detailed description, when taken in conjunction with the accompanying drawings, wherein:

FIGURE 1 is a scanning electron microscope photograph (100x) of representative mixed polymer composite fibers of the invention;
FIGURE 2s a scanning electron microscope photograph (400x) of representative mixed polymer composite fibers of the invention; and
FIGURE 3s a scanning electron microscope photograph (1000x) of representative mixed polymer composite fibers may then be dried.
FIGURE 4 is a diagram of a dry-jet wet process.
FIGURE 5 is a diagram of a centrifugal spinning process.
FIGURE 6 is a diagram of a meltblow spinning process.
FIGURE 7 is a diagram of a meltblow head for the meltblow spinning process.
FIGURE 8 is a diagram of a wet spinning process.

DETAILED DESCRIPTION OF THE INVENTION

The present invention provides a mixed polymer composite fiber and methods for making the mixed polymer composite fiber..

The mixed polymer composite fiber is a fiber comprising a carboxyalkyl cellulose and a galactomannan polymer or a glucomannan polymer. The carboxyalkyl cellulose, which is mainly in the sodium salt form, can be in other salts forms such as potassium and ammonium forms. The mixed polymer composite fiber is formed by intermolecular crosslinking of mixed polymer molecules, and is water insoluble and water-swellable.

In the method, a carboxyalkyl cellulose and a galactomannan polymer or glucomannan polymer are blended in water to provide an aqueous solution; the aqueous solution treated with a first crosslinking agent to provide a gel.

In one embodiment the gel is mixed with a water-miscible solvent to provide fibers; and the fibers treated with a second crosslinking agent to provide crosslinked mixed polymer composite fibers.

In another embodiment the gel is then spun into gel fibers using centrifugal spinning. In another embodiment the gel is extruded into gel fibers using meltblowing. In another embodiment the gel is formed into gel fibers using wet spinning.

In an embodiment the spun or extruded gel fibers are precipitated into solid fibers by being passed into a solvent bath to provide mixed polymer composite fibers. In another embodiment the spun or extruded gel fibers are precipitated into solid fibers by being sprayed with a solvent to provide mixed polymer composite fibers. The solvent bath or spray uses a water miscible solvent.

In another embodiment the bath or spray may contain a second crosslinking agent to provide further crosslinking of the fibers.

The mixed polymer composite fibers may then be dried.

In one aspect, the present invention provides a mixed polymer composite fiber. As used herein, the term "mixed polymer composite fiber" refers to a fiber that is the composite of two different polymer molecules (i.e., mixed polymer molecules). The mixed polymer composite fiber is a homogeneous composition that includes two associated polymers: (1) a carboxyalkyl cellulose and (2) either a galactomannan polymer or a glucomannan polymer.

The carboxyalkyl cellulose useful in making the mixed polymer composite fiber has a degree of carboxyl group substitution (DS) of from about 0.3 to about 2.5. In one embodiment, the carboxyalkyl cellulose has a degree of carboxyl group substitution of from about 0.5 to about 1.5.

Although a variety of carboxyalkyl celluloses are suitable for use in making the mixed polymer composite fiber, in one embodiment, the carboxyalkyl cellulose is carboxymethyl cellulose. In another embodiment, the carboxyalkyl cellulose is carboxyethyl cellulose.

The carboxyalkyl cellulose is present in the mixed polymer composite fiber in an amount from about 60 to about 99% by weight based on the weight of the mixed polymer composite fiber. In one embodiment, the carboxyalkyl cellulose is present in an amount from about 80 to about 95% by weight based on the weight of the mixed polymer composite fiber. In addition to carboxyalkyl cellulose derived from wood pulp containing some carboxyalkyl hemicellulose, carboxyalkyl cellulose derived from non-wood pulp, such as cotton linters, is suitable for preparing the mixed polymer composite fiber. For carboxyalkyl cellulose derived from wood products, the mixed polymer fibers include carboxyalkyl hemicellulose in an amount up to about 20% by weight based on the weight of the mixed polymer composite fiber.

The galactomannan polymer useful in making the mixed polymer composite fiber of the invention can include any one of a variety of galactomannan polymers. In one embodiment, the galactomannan polymer is guar gum. In another embodiment, the galactomannan polymer is locust bean gum. In a further embodiment, the galactomannan polymer is tara gum. In another embodiment, the galactomannan polymer is fenugreek gum.

The glucomannan polymer useful in making the mixed polymer composite fiber of the invention can include any one of a variety of glucomannan polymers. In one embodiment, the glucomannan polymer is konjac gum.

The galactomannan polymer or glucomannan polymer is present in an amount from about 1 to about 20% by weight based on the weight of the mixed polymer composite fiber. In one embodiment, the galactomannan polymer or glucomannan polymer is present in an amount from about 1 to about 15% by weight based on the weight of the mixed polymer composite fiber. In a further embodiment, the galactomannan polymer or glucomannan polymer is present in an amount from about 2 to about 15% by weight based on the weight of the mixed polymer composite fiber.

The preparation of the mixed polymer composite fiber is a multistep process. First, the water-soluble carboxyalkyl cellulose and galactomannan polymer or glucomannan polymer are dissolved in water. Then, a first crosslinking agent is added and mixed to obtain a mixed polymer composite gel formed by intermolecular crosslinking of water-soluble polymers.

Suitable first crosslinking agents include crosslinking agents that are reactive towards hydroxyl groups and carboxyl groups. Representative crosslinking agents include metallic crosslinking agents, such as aluminum (III) compounds, titanium (IV) compounds, bismuth (III) compounds, boron (III) compounds, and zirconium (IV) compounds. The numerals in parentheses in the preceding list of metallic crosslinking agents refers to the valency of the metal.

In one embodiment, the mixed polymer composite fiber is generated by rapid mixing of the mixed polymer composite gel with a water-miscible solvent. This fiber generated after first crosslinking has a high level of sliminess when hydrated and forms soft gels. Therefore this fiber cannot be used in absorbent applications without further treatment. The mixed polymer composite fiber thus obtained is further crosslinked (e.g., surface crosslinked) by treating with a second crosslinking agent in a water-miscible solvent containing water. The composition of water-miscible solvent and water is such that the fiber does not change its fiber form and return to gel state. The second crosslinking agent can be the same as or different from the first crosslinking agent.

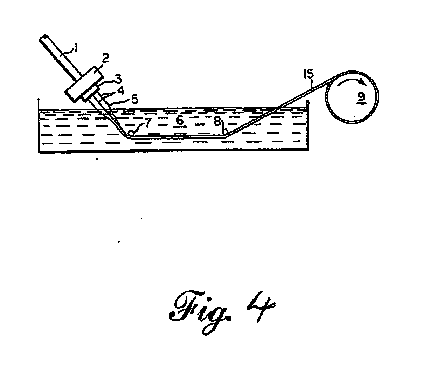

In another embodiment the gel is formed into fibers through the use of a dry-jet wet spinning process. A diagram of the dry jet wet spinning process is shown in Figure 4. The gel is pumped through a transfer line 1 through a spinning block 2, through the orifices of spinneret 3 through a layer of gas or air 5 and into a bath 6 where the fibers 4 are conducted by guides 7 and 8 and precipitated into mixed polymer composite fiber 15 which is wound up on a take-up roll 9.

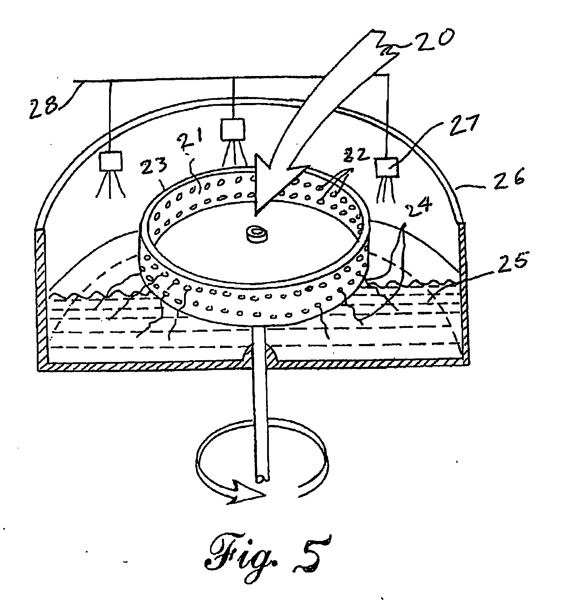

In another embodiment the gel is formed into fibers through the use of centrifugal spinning. Figure 5 is a diagram of centrifugal spinning. In centrifugal spinning the gel 20 is directed in a generally hollow cylinder or drum 21 with a closed base and a multiplicity of small apertures 22 in its sidewalls 23. As the cylinder rotates, the gel is forced out horizontally through the apertures as thin gel strands or gel fibers 24. As the strands meet resistance from the surrounding air they are drawn or stretched. The amount of stretch will depend on readily controllable factors such as cylinder rotational speed, orifice size of the apertures and the viscosity of the gel. The strands either fall by gravity or are forced down by air flow into a water miscible solvent 25 held in a basin 26 where the gel fibers are precipitated into mixed polymer composite fibers. Alternatively, the fibers 24 may be sprayed with a water-miscible solvent from a ring of spray nozzles 27 fed by line 28.

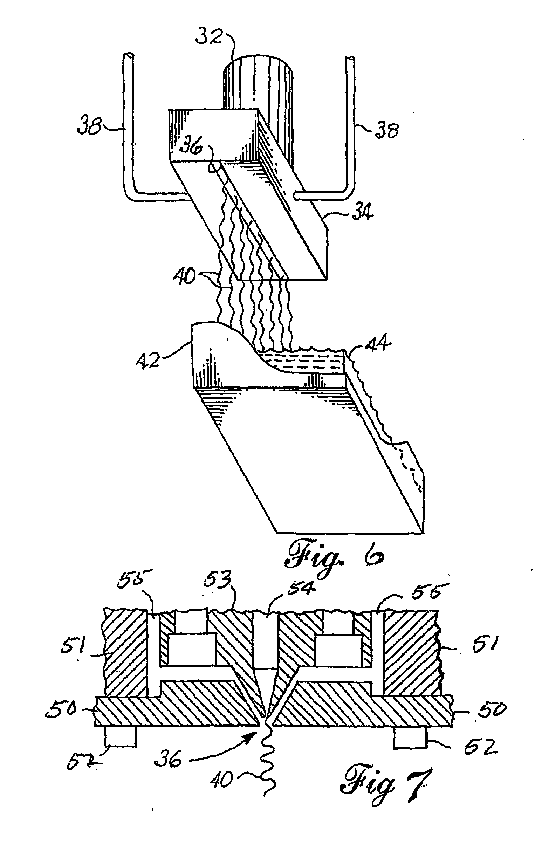

In another embodiment the gel is formed into fibers through the use of melt blowing technology. Figures 6 and 7 are diagrams of melt blowing. In melt blowing the gel is directed to an extruder 32 which forces the gel through an orifice head 34 having a multiplicity of orifices 36. Air or another gas is supplied through lines 38 and surrounds and transports extruded gel fibers 40. The air or gas moves in parallel with the fibers and impinges on the fibers, transporting the fibers, and drawing and stretching the fibers. The gel fibers move into the bath 42 which contains a water-miscible solvent 44 which precipitates the gel fibers to form mixed polymer composite fibers. As in centrifugal spinning the gel fibers may be sprayed with the water-miscible solvent to form the mixed polymer composite fibers instead of being placed in a bath. Below orifice 36 and above bath 42, solvent circulated from bath 42 can be sprayed onto fibers 40 too.

Figure 7 shows a typical extrusion orifice. The orifice plate 50 is bored with a multiplicity of orifices 36. The plate 50 is held to the body of the extrusion head 51 by a series of cap screws 52. An internal member 53 forms the extrusion ports 54 for the gel. It is embraced by air passages 55 that surround the extruded gel fibers 40 causing them to be drawn and to assist in their transport to the bath. The amount the gel fibers are drawn or stretched will depend on the viscosity of the gel, the speed of the fiber travel and the gas travel, and the angle between the gas and the fiber. Depending on the speed and angle of the fiber and gas, long continuous fibers may be formed or short fibers may be formed.

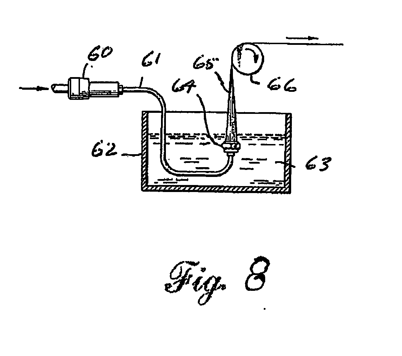

In another embodiment the gel may be formed into fibers by wet spinning. A diagram of wet spinning is shown in Figure 8. In wet spinning the gel is passed by a pump 60 through pipe 61 leading into bath 62 containing the water-miscible solvent 63. The gel is extruded through spinneret 64 directly into the bath to form mixed polymer composite fibers 65 which are guided from by the transfer roll 66 to a take up roll. The amount of time of the gel in the bath will depend on the speed of the fibers and the placement of the spinneret in the bath. A short retention time is shown. A different placement of the spinneret will increase the retention time in the bath. The fibers are fixed in the bath. Alternatively, fiber 65 can be collected on a moving screen.

The mixed polymer composite fiber thus obtained may be further crosslinked (e.g., surface crosslinked) by treating with a second crosslinking agent in the treating bath or spray. The second crosslinking agent can be the same as or different from the first crosslinking agent. The need for a second crosslinking step will depend on the amount of crosslinking that has been generated in the initial crosslinking. If the initial crosslinking is light then the fiber generated after first crosslinking has a high level of sliminess when hydrated and forms soft gels and cannot be used in absorbent applications without further treatment. If the crosslinking in the first or initial crosslinking is greater the fiber generated after the first crosslinking will not be slimy and will be a hard gel.

The mixed polymer fibers of the invention are substantially insoluble in water while being capable of absorbing water. The fibers of the invention are rendered water insoluble by virtue of a plurality of non-permanent intra-fiber metal crosslinks. As used herein, the term "non-permanent intra-fiber metal crosslinks" refers to the nature of the crosslinking that occurs within individual modified fibers of the invention (i.e., intra-fiber) and among and between each fiber's constituent polymer molecules.

The fibers of the invention are intra-fiber crosslinked with metal crosslinks. The metal crosslinks arise as a consequence of an associative interaction (e.g., bonding) between functional groups (e.g., carboxy, carboxylate, or hydroxyl groups) of the fiber's polymers and a multi-valent metal species. Suitable multi-valent metal species include metal ions having a valency of three or greater and that are capable of forming associative interpolymer interactions with functional groups of the polymer molecules (e.g., reactive toward associative interaction with the carboxy, carboxylate, or hydroxyl groups). The polymers are crosslinked when the multi-valent metal species form associative interpolymer interactions with functional groups on the polymers. A crosslink may be formed intramolecularly within a polymer or may be formed intermolecularly between two or more polymer molecules within a fiber. The extent of intermolecular crosslinking affects the water solubility of the composite fibers (i.e., the greater the crosslinking, the greater the insolubility) and the ability of the fiber to swell on contact with an aqueous liquid.

The fibers of the invention include non-permanent intrafiber metal crosslinks formed both intermolecularly and intramolecularly in the population of polymer molecules. As used herein, the term "non-permanent crosslink" refers to the metal crosslink formed with two or more functional groups of a polymer molecule (intramolecularly) or formed with two or more functional groups of two or more polymer molecules (intermolecularly). It will be appreciated that the process of dissociating and re-associating (breaking and reforming crosslinks) the multi-valent metal ion and polymer molecules is dynamic and also occurs during liquid acquisition. During water acquisition the individual fibers and fiber bundles swell and change to gel state. The ability of non-permanent metal crosslinks to dissociate and associate under water acquisition imparts greater freedom to the gels to expand than if the gel was restrictively crosslinked by permanent crosslinks that do not have the ability to dissociate and re-associate. Covalent organic crosslinks, such as ether crosslinks, are permanent crosslinks that do not dissociate and re-associate.

The fibers of the invention have fiber widths of from about 2 $\mu$m to about 50 $\mu$m (or greater) and coarseness that varies from soft to rough.

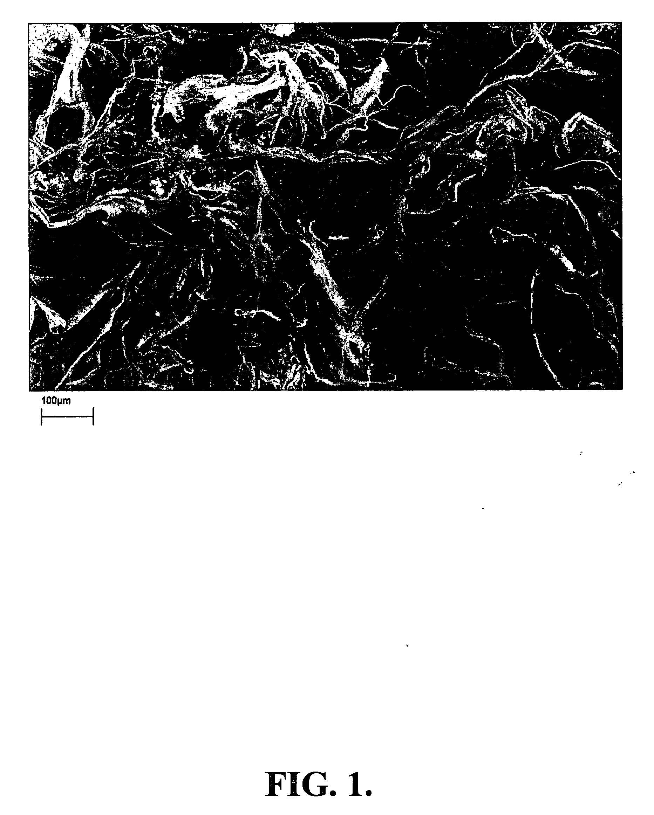

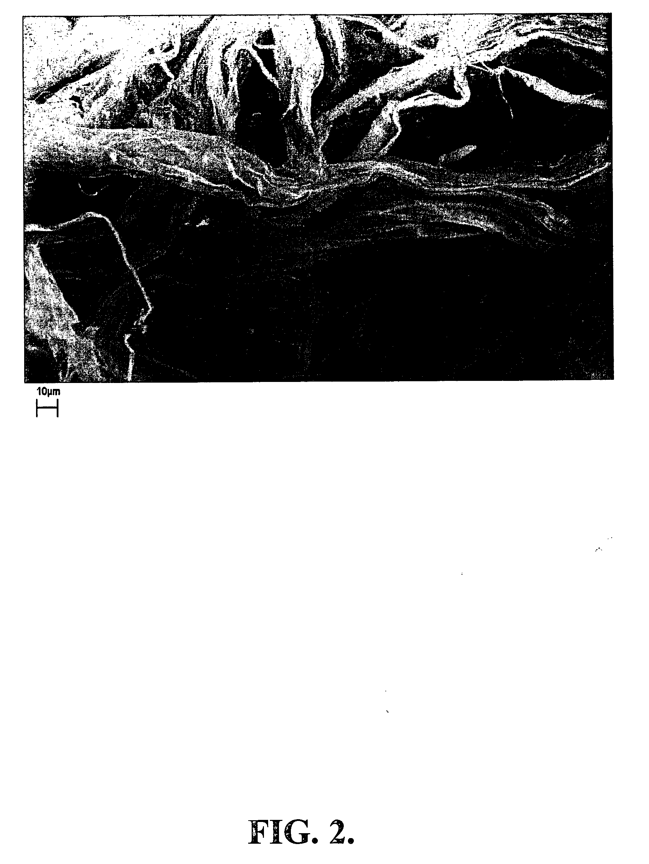

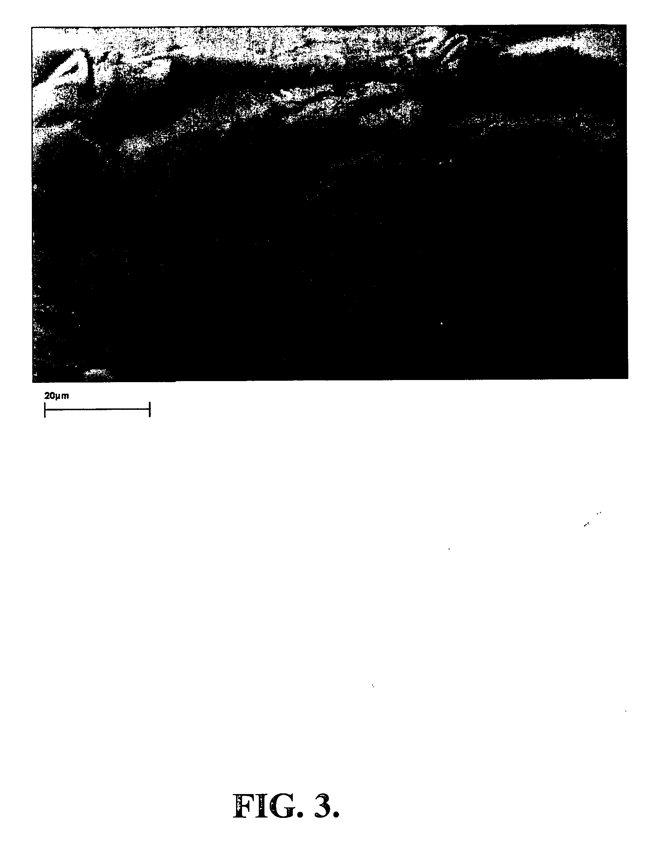

Representative mixed polymer composite fibers of the invention are illustrated in FIGURES 1-3. FIGURE 1 is a scanning electron microscope photograph (100x) of representative mixed polymer composite fibers of the invention (Sample 31, Table 1). FIGURE 2 is a scanning electron microscope photograph (400x) of representative mixed polymer composite fibers of the invention (Sample 31, Table 1). FIGURE 3 is a scanning electron microscope photograph (1000x) of representative mixed polymer composite fibers of the invention (cross-sectional view) (Sample 125, Table 1).

The fibers of the invention are highly absorptive fibers. The fibers have a Free Swell Capacity of from about 30 to about 60 g/g (0.9% saline solution), a Centrifuge Retention Capacity (CRC) of from about 15 to about 35 g/g (0.9% saline solution), and an Absorbency Under Load (AUL) of from about 15 to about 30 g/g (0.9% saline solution).

The fibers of the invention can be formed into pads by conventional methods including air-laying techniques to provide fibrous pads having a variety of liquid wicking characteristics. For example, pads absorb liquid at a rate of from about 10 ml/sec to about 0.005 ml/sec (0.9% saline solution/10 ml application). The integrity of the pads can be varied from soft to very strong.

The mixed polymer composite fibers of the present invention are water insoluble and water swellable. Water insolubility is imparted to the fiber by intermolecular crosslinking of the mixed polymer molecules, and water swellability is imparted to the fiber by the presence of carboxylate anions with associated cations. The fibers are characterized as having a relatively high liquid absorbent capacity for water (e.g., pure water or aqueous solutions, such as salt solutions or biological solutions such as urine). Furthermore, because the mixed polymer fiber has the structure of a fiber, the mixed polymer composite fiber also possesses the ability to wick liquids. The mixed polymer composite fiber of the invention advantageously has dual properties of high liquid absorbent capacity and liquid wicking capacity.

Mixed polymer fibers having slow wicking ability of fluids are useful in medical applications, such as wound dressings and others. Mixed polymer fibers having rapid wicking capacity for urine are useful in personal care absorbent product applications. The mixed polymer fibers can be prepared having a range of wicking properties from slow to rapid for water and 0.9% aqueous saline solutions.

The mixed polymer composite fibers of the invention are useful as superabsorbents in personal care absorbent products (e.g., infant diapers, feminine care products and adult incontinence products). Because of their ability to wick liquids and to absorb liquids, the mixed polymer composite fibers of the invention are useful in a variety of other applications, including, for example, wound dressings, cable wrap, absorbent sheets or bags, and packaging materials.

In one aspect of the invention, methods for making mixed polymer composite fibers are provided.

In one embodiment, the mixed polymer composite fibers are prepared by methods in which the fibers are generated from solution and formed into fibers during the solvent exchange process under shear mixing conditions. As noted above, fiber formation results from shear mixing the gel with the water-miscible solvent and effects solvent exchange and generation of composite fiber in the resultant mixed solvent.

In one embodiment, the method for making the mixed polymer composite fibers (crosslinked fibers) includes the steps of: (a) blending a carboxyalkyl cellulose (e.g., mainly salt form) and a galactomannan polymer or a glucomannan polymer in water to provide an aqueous solution; (b) treating the aqueous solution with a first crosslinking agent to provide a gel; (c) mixing the gel with a water-miscible solvent to provide fibers; and (d) treating the fibers with a second crosslinking agent (e.g., surface crosslinking) to provide mixed polymer composite fibers. The mixed polymer composite fibers so prepared can be fiberized and dried.

In other embodiments of the method, the mixed polymer composite fibers are formed by spinning or extruding the gel into a fiber and then precipitating the fiber to form a mixed polymer composite fiber by a water-miscible solvent bath or spray.

In one embodiment, the method for making the mixed polymer composite fibers (crosslinked fibers) includes the steps of: (a) blending a carboxyalkyl cellulose (e.g., mainly salt form) and a galactomannan polymer or a glucomannan polymer in water to provide an aqueous solution; (b) treating the aqueous solution with a first crosslinking agent to provide a gel; (c) forming a fiber from the gel by centrifugal spinning; and (d) treating the gel fiber in a water-miscible solvent bath or by water-miscible solvent spray to provide to precipitate the mixed polymer composite fibers.

In another embodiment, the method for making the mixed polymer composite fibers (crosslinked fibers) includes the steps of: (a) blending a carboxyalkyl cellulose (e.g., mainly salt form) and a galactomannan polymer or a glucomannan polymer in water to provide an aqueous solution; (b) treating the aqueous solution with a first crosslinking agent to provide a gel; (c) forming a fiber from the gel by melt blowing; and (d) treating the gel fiber in a water-miscible solvent bath or by water-miscible solvent spray to provide mixed polymer composite fibers.

In another embodiment, the method for making the mixed polymer composite fibers (crosslinked fibers) includes the steps of: (a) blending a carboxyalkyl cellulose (e.g., mainly salt form) and a galactomannan polymer or a glucomannan polymer in water to provide an aqueous solution; (b) treating the aqueous solution with a first crosslinking agent to provide a gel; (c) forming a fiber from the gel by wet spinning; and (d) treating the gel fiber in a water-miscible solvent bath to provide mixed polymer composite fibers.

In another embodiment, the method for making the mixed polymer composite fibers (crosslinked fibers) includes the steps of: (a) blending a carboxyalkyl cellulose (e.g., mainly salt form) and a galactomannan polymer or a glucomannan polymer in water to provide an aqueous solution; (b) treating the aqueous solution with a first crosslinking agent to provide a gel; (c) forming a fiber from the gel by jet-dry wet spinning; and (d) treating the gel fiber in a water-miscible solvent bath to provide mixed polymer composite fibers.

In another embodiment step (d) in each of the above methods may include treating the fibers with a second crosslinking agent (e.g., surface crosslinking) by having the second crosslinking agent in the bath or spray to provide mixed polymer composite fibers.

The fibers may have a diameter of 50$\mu$m to 1000 $\mu$m. Melt blown fibers may be nonuniform in diameter along the fiber length.

The mixed polymer composite fibers so prepared can be dried.

In the process, a carboxyalkyl cellulose and a galactomannan polymer or a glucomannan polymer are blended in water to provide an aqueous solution.

Suitable carboxyalkyl celluloses have a degree of carboxyl group substitution of from about 0.3 to about 2.5, and in one embodiment have a degree of carboxyl group substitution of from about 0.5 to about 1.5. In one embodiment, the carboxyalkyl cellulose is carboxymethyl cellulose. The aqueous solution includes from about 60 to about 99% by weight carboxyalkyl cellulose based on the weight of the product mixed polymer composite fiber. In one embodiment, the aqueous solution includes from about 80 to about 95% by weight carboxyalkyl cellulose based on the weight of mixed polymer composite fiber.

Suitable galactomannan polymers include guar gum, locust bean gum, tara gum, and fenugreek gum. Suitable glucomannan polymers include konjac gum. The galactomannan polymer or glucomannan polymer can be from natural sources or obtained from genetically-modified plants. The aqueous solution includes from about 1 to about 20% by weight galactomannan polymer or glucomannan polymer based on the weight of the mixed polymer composite fibers, and in one embodiment, the aqueous solution includes from about 1 to about 15% by weight galactomannan polymer or glucomannan polymer based on the weight of mixed polymer composite fibers.

In the method, the aqueous solution including the carboxyalkyl cellulose and galactomannan polymer or glucomannan polymer is treated with a suitable amount of a first crosslinking agent to provide a gel.

Suitable first crosslinking agents include crosslinking agents that are reactive towards hydroxyl groups and carboxyl groups. Representative crosslinking agents include metallic crosslinking agents, such as aluminum (III) compounds, titanium (IV) compounds, bismuth (III) compounds, boron (III) compounds, and zirconium (IV) compounds. The numerals in parentheses in the preceding list of metallic crosslinking agents refers to the valency of the metal.

Representative metallic crosslinking agents include aluminum sulfate; aluminum hydroxide; dihydroxy aluminum acetate (stabilized with boric acid); other aluminum salts of carboxylic acids and inorganic acids; other aluminum complexes, such as Ultrion 8186 from Nalco Company (aluminum chloride hydroxide); boric acid; sodium metaborate; ammonium zirconium carbonate; zirconium compounds containing inorganic ions or organic ions or neutral ligands; bismuth ammonium citrate; other bismuth salts of carboxylic acids and inorganic acids; titanium (IV) compounds, such as titanium (IV) bis(triethylaminato) bis(isopropoxide) (commercially available from the Dupont Company under the designation Tyzor TE); and other titanates with alkoxide or carboxylate ligands.

The first crosslinking agent is effective for associating and crosslinking the carboxyalkyl cellulose (with or without carboxyalkyl hemicellulose) and galactomannan polymer molecules. The first crosslinking agent is applied in an amount of from about 0.1 to about 20% by weight based on the total weight of the mixed polymer composite fiber. The amount of first crosslinking agent applied to the polymers will vary depending on the crosslinking agent. In general, the fibers have an aluminum content of about 0.04 to about 0.8% by weight based on the weight of the mixed polymer composite fiber for aluminum crosslinked fibers, a titanium content of about 0.10 to about 1.5% by weight based on the weight of the mixed polymer composite fiber for titanium crosslinked fibers, a zirconium content of about 0.09 to about 2.0% by weight based on the weight of the mixed polymer composite fiber for zirconium crosslinked fibers, and a bismuth content of about 0.90 to about 5.0% by weight based on the weight of the mixed polymer composite fiber for bismuth crosslinked fibers.

In one embodiment, the gel formed by treating the aqueous solution of a carboxyalkyl cellulose and a galactomannan polymer with a first crosslinking agent is then mixed with a water-miscible solvent to provide fibers. Suitable water-miscible solvents include water-miscible alcohols and ketones. Representative water-miscible solvents include acetone, methanol, ethanol, isopropanol, and mixtures thereof. In one embodiment, the water-miscible solvent is ethanol. In another embodiment, the water-miscible solvent is isopropanol.

The volume of water-miscible solvent added to the gel ranges from about 1:1 to about 1:5 water (the volume used in making the aqueous solution of carboxyalkyl cellulose and galactomannan polymer) to water-miscible solvent.

In the method, mixing the gel with the water-miscible solvent includes stirring to provide fibers. The mixing step and the use of the water-miscible solvent controls the rate of dehydration and solvent exchange and provides for fiber formation. Mixing can be carried out using a variety of devices including overhead stirrers, Hobart mixers, British disintegrators, and blenders. For these mixing devices, the blender provides the greatest shear and the overhead stirrer provides the least shear. As noted above, fiber formation results from mixing with the water-miscible solvent and effects solvent exchange and dehydration. The nature of fiber produced by the mixing step can be controlled by the type of mixer, rate of mixing, and the percent solids in water (i.e., the amount of carboxyalkyl cellulose and galactomannan polymer present in the aqueous solution prior to addition of the water-miscible solvent).

For 1% solids in water, overhead mixers and stirrers including, for example, spiral mixers, provide relatively coarse fibers. These fibers may have the form of shredded paper. Fine fibers are produced using high shear devices, such as a blender (high speed Waring blender). These fine fibers have the appearance of disintegrated cotton fibers. In use, coarse fibers are advantageous for wicking and for avoiding gel blocking during water acquisition and change of fiber form to gel form. Fine fibers are subject to gel blocking, which results from fibers swelling and the collapse of interstitial channels useful for liquid wicking during water acquisition and change of fiber form to gel form.

For 2% solids in water, overhead mixers and stirrers provide fewer coarse fibers than in the 1% solids in water, and high shear devices, such as a blender, produce a fine fiber that is relatively more coarse than that produced in the 1% solids in water.

For 4% solids in water, relatively higher shear devices, such as a blender, produce fine fibers that are relatively more coarse than the fine fibers produced in the 1 % solids in water.

Increasing percent solids in water beyond 4% may require an increase in temperature to achieve fiber formation. Percent solids in water greater than 4% are advantageous for increased throughput and therefore lower cost of production.

In one embodiment, mixing the gel with a water-miscible solvent to provide fibers comprises mixing a 1 or 2% solids in water with an overhead mixer or stirrer. In another embodiment, mixing the gel with a water-miscible solvent to provide fibers comprises mixing 4% solids in water with a blender. For large scale production alternative mixing equipment with suitable mixing capacities are used.

Fibers formed from the mixing step are treated with a second crosslinking agent in a mixture of water and a water miscible solvent in suitable proportions so that the fibers do not lose their fiber form and form a gel. The resultant crosslinked fibers (e.g., surface crosslinked fibers) are then washed with a water-miscible solvent and air dried or oven dried below 80 °C to provide the mixed polymer composite fibers.

In another embodiment, the gel formed by treating the aqueous solution of a carboxyalkyl cellulose and a galactomannan polymer with a first crosslinking agent is then spun or extruded into a gel fiber by centrifugal spinning, meltblowing or wet spinning.

The spun or extruded gel fibers are then precipitated to form mixed polymer composite fibers by treatment by a water-miscible solvent in either a bath or spray.

Suitable water-miscible solvents include water-miscible alcohols and ketones. Representative water-miscible solvents include acetone, methanol, ethanol, isopropanol, and mixtures thereof. In one embodiment, the water-miscible solvent is ethanol. In another embodiment, the water-miscible solvent is isopropanol.

If the fibers formed from the spinning or extrusion step are treated in a mixture of water and a water miscible solvent, the proportions of water and solvent must be such that the fibers do not lose their fiber form and form a gel.

A second crosslinking agent may be used in the bath or spray.

The resultant fibers, either with one crosslinking agent or surface crosslinked with a second crosslinking agent, are then washed with a water-miscible solvent and air dried or oven dried below 80 °C to provide the mixed polymer composite fibers.

The second crosslinking agent is effective in further crosslinking (e.g., surface crosslinking) the mixed polymer composite fibers. Suitable second crosslinking agents include crosslinking agents that are reactive towards hydroxyl groups and carboxyl groups. The second crosslinking agent can be the same as or different from the first crosslinking agent. Representative second crosslinking agents include the metallic crosslinking agents noted above useful as the first crosslinking agents.

The second crosslinking agent can be applied at a relatively higher level than the first crosslinking agent per unit mass of fiber. This provides a higher degree of crosslinking on the surface of the fiber relative to the interior of the fiber. As described above, metal crosslinking agents form crosslinks between carboxylate anions and metal atoms or hydroxyl oxygen and metal atoms. These crosslinks can migrate from one oxygen atom to another when the mixed polymer fiber absorbs water and forms a gel. However, having a higher level of crosslinks on the surface of the fiber relative to the interior provides a superabsorbent fiber with a suitable balance in free swell, centrifuge retention capacity, absorbency under load for aqueous solutions and lowers the gel blocking that inhibits liquid transport.

The second crosslinking agent is applied in an amount from about 0.1 to about 20% by weight based on the total weight of mixed polymer composite fibers. The amount of second crosslinking agent applied to the polymers will vary depending on the crosslinking agent. The product fibers have an aluminum content of about 0.04 to about 2.0% by weight based on the weight of the mixed polymer composite fiber for aluminum crosslinked fibers, a titanium content of about 1.0 to about 4.5% by weight based on the weight of the mixed polymer composite fiber for titanium crosslinked fibers, a zirconium content of about 0.09 to about 6.0% by weight based on the weight of the mixed polymer composite fiber for zirconium crosslinked fibers; and a bismuth content of about 0.09 to about 5.0% by weight based on the weight of the mixed polymer composite fiber for bismuth crosslinked fibers.

The second crosslinking agent may be the same as or different from the first crosslinking agent. Mixtures of two or more crosslinking agents in different ratios may be used in each crosslinking step.

The preparation of representative mixed polymer composite fibers of the invention are described in Examples 1-8.

The absorbent properties of the representative mixed polymer composite fibers are summarized in the Table 1. In Table 1, "CMC 9H4F" refers to a carboxymethyl cellulose commercially available from Hoechst Celanese under that designation; "PA-CMC" refers to CMC made from northern softwood pulp; "LV-PN" refers to CMC made from west coast pine pulp; "LV-HW" refers to CMC made from west coast hardwood pulp; "LV-FIR" refers to CMC made from douglas fir pulp; and "KL-SW" refers to CMC made from northern softwood pulp; "i-PrOH" refers to isopropanol; "w wash" refers to washing the treated fibers with 100% ethanol or 100% isopropanol before drying; and "wo washing" refers to the process in which the treated fibers are not washed before drying.

The metal analysis for select representative mixed polymer composite fibers is summarized in the Table 2. Samples 1A, 2A, 3A and 4A refer to Samples 1, 2, 3, and 4, respectively, without treatment with a second crosslinking agent.

In Tables 1 and 2, "% wgt total wgt, applied" refers to the amount of first crosslinking agent applied to the total weight of CMC and guar gum; "Second crosslinking agent/2g" refers to the amount of second crosslinking agent applied per 2 g first crosslinked product; "BA" refers to boric acid, and "EtOH" refers to ethanol.

Test Methods

Free Swell and Centrifuge Retention Capacities

The materials, procedure, and calculations to determine free swell capacity (g/g) and centrifuge retention capacity (CRC) (g/g) were as follows.

Test Materials:

Japanese pre-made empty tea bags (available from Drugstore.com, IN PURSUIT OF TEA polyester tea bags 93 mm x 70 mm with fold-over flap. (http:www.mesh.ne.jp/tokiwa/).
Balance (4 decimal place accuracy, 0.0001 g for air-dried superabsorbent polymer (ADS SAP) and tea bag weights); timer; 1% saline; drip rack with clips (NLM 211); and lab centrifuge (NLM 211, Spin-X spin extractor, model 776S, 3,300 RPM, 120v).

Test Procedure:

1. Determine solids content of ADS.
2. Pre-weigh tea bags to nearest 0.000 1 g and record.
3. Accurately weigh 0.2025g +/- 0.0025g of test material (SAP), record and place into pre-weighed tea bag (air-dried (AD) bag weight). (ADS weight + AD bag weight = total dry weight).
4. Fold tea bag edge over closing bag.
5. Fill a container (at least 3 inches deep) with at least 2 inches with 1 % saline.
6. Hold tea bag (with test sample) flat and shake to distribute test material evenly through bag.
7. Lay tea bag onto surface of saline and start timer.
8. Soak bags for specified time (e.g., 30 minutes).
9. Remove tea bags carefully, being careful not to spill any contents from bags, hang from a clip on drip rack for 3 minutes.
10. Carefully remove each bag, weigh, and record (drip weight).
11. Place tea bags onto centrifuge walls, being careful not to let them touch and careful to balance evenly around wall.
12. Lock down lid and start timer. Spin for 75 seconds.
13. Unlock lid and remove bags. Weigh each bag and record weight (centrifuge weight).

Calculations:

The tea bag material has an absorbency determined as follows:

Free Swell Capacity, factor = 5.78
Centrifuge Capacity, factor = 0.50
Z = Oven dry SAP wt (g)/Air dry SAP wt (g)

Free Capacity (g/g):

$$\frac{[(\text{drip wt (g)} - \text{dry bag wt (g)}) - (\text{AD SAP wt (g)})] - (\text{dry bag wt (g)} * 5.78)}{(\text{AD SAP wt (g)} * Z)}$$

Centrifuge Retention Capacity (g/g):

$$\frac{[\text{centrifuge wt (g)} - \text{dry bag wt (g)} - (\text{AD SAP wt (g)})] - (\text{dry bag wt(g)} * 0.50)}{(\text{AD SAP wt} * Z)}$$

Absorbency Under Load (AUL)

The materials, procedure, and calculations to determine AUL were as follows.

Test Materials:

Mettler Toledo PB 3002 balance and BALANCE-LINK software or other compatible balance and software. Software set-up: record weight from balance every 30 sec (this will be a negative number. Software can place each value into EXCEL spreadsheet.

Kontes 90 mm ULTRA-WARE filter set up with fritted glass (coarse) filter plate. clamped to stand; 2 L glass bottle with outlet tube near bottom of bottle; rubber stopper with glass tube through the stopper that fits the bottle (air inlet); TYGON tubing; stainless steel rod/plexiglass plunger assembly (71mm diameter); stainless steel weight with hole drill through to place over plunger (plunger and weight = 867 g); VWR 9.0 cm filter papers (Qualitative 413 catalog number 28310-048) cut down to 80 mm size; double-stick SCOTCH tape; and 0.9% saline.

Test Procedure:

1. Level filter set-up with small level.
2. Adjust filter height or fluid level in bottle so that fritted glass filter and saline level in bottle are at same height.
3. Make sure that there are no kinks in tubing or air bubbles in tubing or under fritted glass filter plate.
4. Place filter paper into filter and place stainless steel weight onto filter paper.
5. Wait for 5-10 min while filter paper becomes fully wetted and reaches equilibrium with applied weight.
6. Zero balance.
7. While waiting for filter paper to reach equilibrium prepare plunger with double stick tape on bottom.
8. Place plunger (with tape) onto separate scale and zero scale.
9. Place plunger into dry test material so that a monolayer of material is stuck to the bottom by the double stick tape.
10. Weigh the plunger and test material on zeroed scale and record weight of dry test material (dry material weight 0.15 g +/- 0.05 g).
11. Filter paper should be at equilibrium by now, zero scale.
12. Start balance recording software.
13. Remove weight and place plunger and test material into filter assembly.
14. Place weight onto plunger assembly.
15. Wait for test to complete (30 or 60 min)
16. Stop balance recording software.

Calculations:

A = balance reading (g) * -1 (weight of saline absorbed by test material)
B = dry weight of test material (this can be corrected for moisture by multiplying the AD weight by solids %).

AUL (g/g) = AB (g 1 % saline/1 g test material)

The following examples are provided for the purpose of illustrating, not limiting, the invention.

EXAMPLES

Example 1

The Preparation of Representative Mixed Polymer Composite Fibers: Aluminum Sulfate and Boric Acid/Aluminum Sulfate and Boric Acid Crosslinking

In this example, the preparation of representative mixed polymer composite fibers crosslinked with aluminum sulfate/boric acid and aluminum sulfate is described.

A solution of CMC 9H4F 10.0 g OD in 900 ml deionized (DI) water was prepared with vigorous stirring to obtain a CMC solution. Guar gum (0.6 g) was dissolved in 50 ml DI water and mix well with the CMC solution. The solution was stirred for one hour to allow complete mixing of the two polymers.

The polymer mixture was blended in the blender for 5 minutes. Fully dissolve boric acid 0.1 g in 30 ml DI water. Weigh 0.6 g aluminum sulfate octadecahydrate and dissolve in 20 ml DI water. Transfer boric acid solution and aluminum sulfate solution to the polymer solution and blend for 5 minutes to mix to provide a gel. Leave the gel at ambient temperature (25 C) for one hour. Transfer the gel into a large plastic beaker with 2 liters of denatured ethanol and stir for one hour using an overhead stirrer. Filter the precipitate and place in 1 liter dry denatured ethanol for one hour. Filter the precipitate and air dry.

Dissolve 0.3 g of boric acid and 0.75 g of aluminum sulfate octadecahydrate in 150 ml of deionized water and mix with 450 ml of denatured ethanol. To the stirred solution add 6.0 g of fiber, prepared as described above, and leave for 20 minutes at 25°C. Filter the fiber and press free of excess solution. Air dry the resulting product fiber at 25°C. Free swell (59.39 g/g), centrifuge retention capacity (32.8 g/g), AUL at 0.3 psi (28.22 g/g) for 0.9% saline solution.

Example 2

The Preparation of Representative Mixed Polymer Composite Fibers: Aluminum Sulfate and Boric Acid/Aluminum Sulfate and Boric Acid Crosslinking

In this example, the preparation of representative mixed polymer composite fibers crosslinked with aluminum sulfate/boric acid and aluminum sulfate/boric acid is described. A solution of CMC 9H4F (5.0 g OD) in 450 ml deionized water was prepared with vigorous stirring to obtain a CMC solution. Guar gum (0.3 g) was dissolved in 25 ml DI water and mixed with the CMC solution. The solution was stirred for one hour to allow complete mixing of the two polymers.

The polymer mixture was blended in the blender for 5 minutes. Fully dissolve boric acid 0.05 g in 15 ml DI water. Weigh 0.2 g aluminum sulfate octadecahydrate and dissolve in 10 ml DI water. Transfer boric acid solution and aluminum sulfate solution to the polymer solution and blend for 5 minutes to mix well. Leave the gel at ambient temperature (25°C) for one hour. Transfer the gel into a disintegrator with 1.5 liters of denatured ethanol. Mix for 5 minutes (blades round and dull to avoid fiber damage and 2 rev/ sec) and filter the precipitate. To 400 ml of the filtrate add 50 ml aqueous solution containing 0.25 g of boric acid and 0.7 g of aluminum sulfate octadecahydrate. Add the fiber back into the crosslinking solution. Allow crosslinking to continue for 20 minutes. Filter and place the fiber in 500 ml of denatured ethanol and mix for 15 minutes. Filter the product fiber and dry in an oven at 50°C for 15 minutes and then air dry at 25°C with fluffing. Free swell (55.63 g/g), centrifuge retention capacity (23.63 g/g), AUL at 0.3 psi (32.02 g/g) for 0.9% saline solution.

Example 3

The Preparation of Representative Mixed Polymer Composite Fibers: Aluminum Sulfate/Aluminum Sulfate Crosslinking

In this example, the preparation of representative mixed polymer composite fibers crosslinked with aluminum sulfate and aluminum sulfate is described.

A solution of CMC 9H4F (5.0 g OD) in 450 ml deionized water was prepared with vigorous stirring to obtain a CMC solution. Guar gum (0.3 g) was dissolved in 25 ml DI water and mixed with the CMC solution. The solution was stirred for one hour to allow complete mixing of the two polymers.

The polymer mixture was blended in the blender for 5 minutes. Weigh 0.3 g aluminum sulfate octadecahydrate and dissolve in 25 ml DI water. Transfer aluminum sulfate solution to the polymer solution and blend for 5 minutes to mix well. Leave the gel at ambient temperature (25°C) for one hour. Transfer the gel into a Hobart type blender with 1.5 liters of denatured ethanol. Mix for 15 minutes (anchor type blades) and filter the precipitate. To 400 ml of the filtrate add 50 ml aqueous solution containing 0.75 g of aluminum sulfate octadecahydrate. Add the fiber back into the crosslinking solution. Allow the crosslinking to continue for 20 minutes. Filter and place the fiber in 500 ml of denatured ethanol and mix for 15 minutes. Filter the product fiber and dry in an oven at 50°C for 15 minutes and then air dry at 25°C with fluffing. Free swell (56.35 g/g), centrifuge retention capacity (32.8 g/g), AUL at 0.3 psi (29.35 g/g) for 0.9% saline solution.

Example 4

The Preparation of Representative Mixed Polymer Composite Fibers: Aluminum Sulfate and Boric Acid/Aluminum Sulfate Crosslinking

In this example, the preparation of representative mixed polymer composite fibers crosslinked with aluminum sulfate/boric acid and aluminum sulfate is described.

A solution of CMC 9H4F (10.0 g OD) in 900 ml deionized water was prepared with vigorous stirring to obtain a CMC solution. Guar gum (0.6 g) was dissolved in 50 ml DI water and mix well with the CMC solution. The solution was stirred for one hour to allow complete mixing of the two polymers.

The polymer mixture was blended in the blender for 5 minutes. Weigh 0.4 g aluminum sulfate octadecahydrate and 0.1 g boric acid and dissolve in 50 ml DI water. Transfer aluminum sulfate and boric acid solution to the polymer solution and blend for 5 minutes to mix well. Leave the gel at ambient temperature (25°C) for one hour. Transfer the gel into a Waring type blender with one liter of isopropanol. Mix for 1 minute at low speed (gave a softer gel). Transfer the gel to a 5 gallon plastic bucket. Add two liters of isopropanol and mix rapidly with the vertical spiral mixer for 30 minutes. Filter and place the fiber in 500 ml of isopropanol and leave for 15 minutes. Filter the fiber and dry in an oven at 66°C for 15 - 30 minutes.

Dissolve 0.32 g of aluminum sulfate octadecahydrate in 100 ml of deionized water and mix with 300 ml of denatured ethanol. To the stirred solution add 2.0 g fiber, prepared as described above, and leave for 30 minutes at 25°C. Filter the SAP and press excess solution out of the SAP. Filter and dry the product fiber at 66°C for 15 minutes in an oven. Free swell (67.09 g/g), centrifuge retention capacity (33.28 g/g), AUL at 0.3 psi (29.02 g/g) for 0.9% saline solution.

Example 5

The Preparation of Representative Mixed Polymer Composite Fibers: Aluminum Sulfate/Aluminum Sulfate Crosslinking

In this example, the preparation of representative mixed polymer composite fibers crosslinked with aluminum sulfate and aluminum sulfate is described.

A solution of CMC 9H4F (10.0 g OD) in 900 ml deionized water was prepared with vigorous stirring to obtain a solution. Guar gum (0.6 g) was dissolved in 50 ml DI water and mixed well with the CMC solution. The solution was stirred for one hour to allow complete mixing of the two polymers.

The polymer mixture was blended in the blender for 5 minutes. Weigh 0.4 g aluminum sulfate octadecahydrate and dissolve in 50 ml DI water. Transfer aluminum sulfate solution to the polymer solution and blend for 5 minutes to mix well. Leave the gel at ambient temperature (25°C) for one hour. Transfer the gel into a Waring type blender with one liter of isopropanol. Mix for 1 minute at low speed (gave a softer gel). Transfer the gel to a 5 gallon plastic bucket. Add two liters of isopropanol and mix rapidly with the vertical spiral mixer for 30 minutes. Filter and place the fiber in 500 ml of isopropanol and leave for 15 minutes. Filter the fiber and dry in an oven at 66°C.

Dissolve 0.34 g of aluminum sulfate octadecahydrate in 100 ml of deionized water and mix with 300 ml of denatured ethanol. To the stirred solution add 2.0 g of fiber, prepared as described above, and leave for 30 minutes at 25°C. Filter the fiber and press excess solution. Filter and dry the product fiber at 66°C for 15 minutes in an oven. Free swell (63.53 g/g), centrifuge retention capacity (28.58 g/g), AUL at 0.3 psi (22.15 g/g) for 0.9% saline solution.

Example 6

The Preparation of Representative Mixed Polymer Composite Fibers: Ammonium Zirconium Carbonate/Aluminum Sulfate Crosslinking

In this example, the preparation of representative mixed polymer composite fibers crosslinked with ammonium zirconium carbonate and aluminum sulfate is described.

A solution of CMC 9H4F (10.0 g OD) in 900 ml deionized water was prepared with vigorous stirring to obtain a smooth solution. Guar gum (0.6 g) was dissolved in 50 ml DI water and mixed well with the CMC solution. The solution was stirred for one hour to allow complete mixing of the two polymers.

The polymer mixture was blended using a kitchen blender for 5 minutes. Weigh 0.5 g of ammonium zirconium carbonate solution in water (15% $ZrO_2$ and dissolve in 50 ml DI water. Transfer the ammonium zirconium carbonate solution to the polymer solution and blend for 5 minutes. Heat the gel at 75°C for 2 hours. Transfer the gel into a Waring type blender with one liter of isopropanol. Mix for one minute at low speed to form a softer gel. Transfer the gel into 5 gallon plastic bucket. Add two liters of isopropanol and mix rapidly with the vertical spiral mixer for 30 minutes. Filter and place the fiber in 500 ml of isopropanol and stir for 15 minutes. Filter and dry the fiber in an oven at 66°C for 15 minutes.

Dissolve 0.16 g of aluminum sulfate octadecahydrate in 25 ml DI water and mix with 75 ml of isopropanol. To the solution add 1.0 g of fiber, prepared as described above, and stir for 30 minutes at 25°C. Filter and dry the product fiber in an oven at 66°C for 15 minutes. Free swell (45.01 g/g), centrifuge retention capacity (22.73 g/g), AUL at 0.3 psi (23.06 g/g) for 0.9% saline solution.

Example 7

The Preparation of Representative Mixed Polymer Composite Fibers: Bismuth Ammonium Citrate/Aluminum Sulfate Crosslinking

In this example, the preparation of representative mixed polymer composite fibers crosslinked with bismuth ammonium citrate and aluminum sulfate is described.

A solution of CMC 9H4F (10.0 g OD) in 900 ml deionized water was prepared with vigorous stirring to obtain a solution. Guar gum (0.6 g) was dissolved in 50 ml DI water and mixed well with the CMC solution. The solution was stirred for one hour to allow complete mixing of the two polymers.

The polymer mixture was heated at 80°C for 45 minutes and then blended using a kitchen blender for 5 minutes. Weigh 0.4 g of bismuth ammonium citrate and dissolve in 50 ml of DI water. Transfer the bismuth ammonium citrate suspension to the polymer solution and blend for 5 minutes. Heat the gel at 80°C for 2 hours. Transfer the gel into a Waring type blender with one liter of isopropanol. Mix for one minute at low speed to form a softer gel. Transfer the gel into 5 gallon plastic bucket. Add two liters of isopropanol and mix rapidly with the vertical spiral mixer for 30 minutes. Filter and place the fiber in 500 ml of isopropanol and stir for 15 minutes. Filter the fiber and pass through two times through a fluffer. Dry the fiber in an oven at 66°C for 15 minutes.

Dissolve 0.16 g of aluminum sulfate octadecahydrate in 25 ml DI water and mix with 75 ml of isopropanol. To the solution add 1.0 g of fiber, prepared as described above, and stir for 30 minutes at 25°C. Filter and dry the product fiber in an oven at 66°C for 15 minutes. Free swell (55.22 g/g), centrifuge retention capacity (24.00 g/g) for 0.9% saline solution.

Example 8

The Preparation of Representative Mixed Polymer Composite Fibers: Aluminum Sulfate/Aluminum Sulfate Crosslinking

In this example, the preparation of representative mixed polymer composite fibers crosslinked with aluminum sulfate and aluminum sulfate is described.

A solution of 0.94 DS Kamloops softwood CMC (10.0 g OD) in 900 ml deionized water was prepared with vigorous stirring to obtain a solution. Guar gum (0.6 g) was dissolved in 50 ml DI water and mixed well with the CMC solution. The solution was stirred for one hour to allow complete mixing of the two polymers.

The polymer mixture was blended in the blender for 5 minutes. Weigh 0.8 g aluminum sulfate octadecahydrate 50 ml DI water. Transfer the aluminum sulfate solution to the polymer solution and blend for 5 minutes to mix well. Leave the gel at ambient temperature (25°C) for one hour. Transfer the gel to a 5 gallon plastic bucket. Add three liters of ethanol and mix rapidly with the vertical spiral mixer for 30 minutes. Filter and place the fiber in one liter of ethanol and stir for 15 minutes. Filter the fiber and dry in an oven at 66°C for 15-30 minutes with fluffing.

Dissolve 1.12 g of aluminum sulfate octadecahydrate in 175 ml of deionized water and mix with 525 ml of denatured ethanol. To the stirred solution add 7.0 g of fiber, prepared as described above, and leave for 30 minutes at 25°C. Filter the product fiber and press excess solution out of the product fiber. Filter and dry the fiber at 66°C for 15 minutes in an oven. Free swell (51.82 g/g), centrifuge retention capacity (19.55 g/g), AUL at 0.3 psi (23.24 g/g) for 0.9% saline solution.

Table 1. Composition and Absorbent Properties of Precipitated Superabsorbent Fiber From Crosslinked Aqueous Mixtures of CMC and Galactomannans

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 1 | CMC 9H4F | 5.4 | $Al_2(SO_4)_3$ 2.72 %, B$(OH)_3$ 0.9% | 0.1 g BA and 0.125g $Al_2(SO_4)_3$ | EtOH | 59.39 | 32.8 | 28.22 |
| 2 | CMC 9H4F | 5.4 | $Al_2(SO_4)_3$ 1.83%, B$(OH)_3$ 0.9% | 0.1 g BA and 0.125g $Al_2(SO_4)_3$ | EtOH | 64.96 | 39.85 | 28.88 |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 3 | CMC 9H4F | 5.4 | $Al_2(SO_4)_3$ 0.9%, $B(OH)_3$ 0.9% | 0.1g BA and 0.125g $Al_2(SO_4)_3$ | EtOH | 64.76 | 38.73 | 27.86 |
| 4 | CMC 9H4F | 5.4 | $Al_2(SO_4)_3$ 2.75% | 0.1g BA and 0.125g $Al_2(SO_4)_3$ | EtOH | 57.2 | 32.71 | 29.51 |
| 5 | CMC 9H4F | 5.4 | $Al_2(SO_4)_3$ 2.72%, B(OH)a 0.9% | 0.1g BA and 0.125g $Al_2(SO_4)_3$ | EtOH | 53.45 | 19.07 | 37.95 |
| 6 | CMC 9H4F CMC 9H4F | 5.4 | $Al_2(SO_4)_3$ 1.83%. $B(OH)_3$ 0.9% | 0.1g BA and 0.125g $Al_2(SO_4)_3$ | EtOH | 57.84 | 32.51 | 29.91 |
| 7 | CMC 9H4F CMC9H4F | 5.4 | $Al_2(SO_4)_3$ 0.9%. $B(OH)_3$ 0.9% | 0.1g BA and 0.125g $Al_2(SO_4)_3$ | EtOH | 57.18 | 32.06 | 34.24 |
| 8 | CMC 9H4F | 5.4 | $Al_2(SO_4)_3$ 2.75% | 0.1g BA and 0.125g $Al_2(SO_4)_3$ | EtOH | 55.2 | 25.4 | 31.84 |
| 9 | CMC 9H4F | 5.4 | $Al_2(SO_4)_3$ 2.72%, $B(OH)_3$ 0.9% | 0.1g BA and 0.15g $Al_2(SO_4)_3$ | EtOH | 52.85 | 27.88 | 33.84 |
| 10 | CMC 9H4F | 5.4 | $Al_2(SO_4)_3$ 1.83%, $B(OH)_3$ 0.9% | 0.1g BA and 0.15g $Al_2(SO_4)_3$ | EtOH | 48.05 | 23.49 | 33.46 |
| 11 | CMC 9H4F | 5.4 | $Al_2(SO_4)_3$ 0.9%, B(OH)3 0.9 % | 0.1g BA and 0.15g $Al_2(SO_4)_3$ | EtOH | 51.16 | 24.14 | 28.02 |
| 12 | CMC 9H4F | 5.4 | $Al_2(SO_4)_3$ 2.75% | 0.1g BA and 0.15g $Al_2(SO_4)_3$ | EtOH | 45.65 | 22.12 | 27.55 |
| 13 | CMC 9H4F | 5.4 | $Al_2(SO_4)_3$ 1.83%, $B(OH)_3$ 0.9% | 0.1g BA and 0.125g $Al_2(SO_4)_3$ | EtOH | 55.75 | 29.61 | 31.95 |
| 14 | CMC 9H4F | 5.4 | $Al_2(SO_4)_3$ 1.83%, $B(OH)_3$ 0.9% | 0.1g BA and 0.15g $Al_2(SO_4)_3$ | EtOH | 51.01 | 19.46 | 26.11 |
| 15 | CMC 9H4F | 5.4 | $Al_2(SO_4)_3$ 2.75% | 0.1g BA and 0.125g $Al_2(SO_4)_3$ | EtOH | 51.09 | 27.93 | 29.57 |
| 16 | CMC 9H4F | 5.4 | $Al_2(SO_4)_3$ 2.75% | 0.1g BA and 0.15g $Al_2(SO_4)_3$ | EtOH | 49.69 | 23.12 | 27.8 |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 17 | CMC 9H4F | 5.4 | $Al_2(SO_4)_3$ 1.83%, $B(OH)_3$ 0.9% | 0.1g BA and 0.125g $Al_2(SO_4)_3$ | EtOH | 61.98 | 38.07 | 28.48 |
| 18 | CMC 9H4F | 5.4 | $Al_2(SO_4)_3$ 0.9%, $B(OH)3$ 0.9% | 0.1g BA and 0.15g $Al_2(SO_4)_3$ | EtOH | 61.85 | 39.62 | 28.8 |
| 19 | CMC 9H4F | 5.4 | $Al_2(SO_4)_3$2.72%, $B(OH)_3$ 0.9% | 0.1g BA and 0.125g $Al_2(SO_4)_3$ | EtOH | 42.32 | 24.64 | 21.44 |
| 20 | CMC 9H4F CMC 9H4F | 5.4 | $Al_2(SO_4)_3$ 2.72 %, $B(OH)_3$ 0.9% | 0.1g BA and 0.125g $Al_2(SO_4)_3$ | EtOH | 54.17 | 29.68 | 26.4 |
| 21 | CMC 9H4F | 5.4 | $Al_2(SO_4)_3$ 1.83%, $B(OH)3$ 0.9% | 0.1g BA and 0.125g $Al_2(SO_4)_3$ | EtOH | 47.96 | 27.92 | 24.24 |
| 22 | CMC 9H4F | 5.4 | $Al_2(SO_4)_3$ 1.83%, $B(OH)_3$ 0.9% | 0.1g BA and 0.125g Ab$(SO_4)_3$ | EtOH | 49.97 | 26.34 | 24.33 |
| 23 | CMC 9H4F | 5.4 | $Al_2(SO_4)_3$1.83%, $B(OH)_3$ 0.9% | 0.1g BA and 0.15g $Al_2(SO_4)_3$ (15 min) | EtOH | 56.32 | 28.71 | 31.44 |
| 24 | CMC 9H4F | 5.4 | $Al_2(SO_4)_3$ 1.83 %, $B(OH)_3$ 0.9% | 0.1g BA and 0.125g $Al_2(SO_4)_3$ (30min) | EtOH | 54.17 | 26 12 | 33 17 |
| 25 | CMC 9H4F | 5.4 | $Al_2(SO_4)_3$ 1.83%, $B(OH)_3$ 0.9% | 0.1g BA and 0.125g $Al_2(SO_4)_3$ (45 min) | EtOH | 56.1 | 26.73 | 38.84 |
| 26 | CMC 9H4F | 5.4 | $Al_2(SO_4)_3$ 1.83 %, $B(OH)_3$ 0.9% | 0.1g BA and 0.125g $Al_2(SO_4)_3$ (1 hr) | EtOH | 54.66 | 27.8 | 35.15 |
| 27 | CMC 9H4F | 5.4 | $Al_2(SO_4)_3$ 1.83%, $B(OH)_3$ 0.9% | $Al_2(SO_4)_3$ (20 min) $Al_2(SO_4)_3$ (20 min) | EtOH | 58.49 | 28.89 | 32.88 |
| 28 | CMC 9H4F | 5.4 | $Al_2(SO_4)_3$ 1.83 %, $B(OH)_3$ 0.9% | 0.1g BA and 0.14g $Al_2(SO_4)_3$ (20 min) | EtOH | 54.43 | 23.89 | 30.8 |
| 29 | CMC 9H4F | 5.4 | $Al_2(SO_4)_3$ 1.83 %, $B(OH)_3$ 0.9% | 0.1g BA and 0.15g $Al_2(SO_4)_3$ (20 min) | EtOH | 52.22 | 23.47 | 37.91 |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 30 | CMC 9H4F | 5.4 | $Al_2(SO_4)_3$ 1.83 %, $B(OH)_3$ 0.9% | 0.1 g BA and 0.15g $Al_2(SO_4)_3$ (20 min) | EtOH | 51.35 | 20.37 | 33.6 |
| 31 | CMC 9H4F | 5.4 | $Al_2(SO_4)_3$ 1.83 %, $B(OH)_3$ 0.9% | 0.1 g BA and 0.14g $Al_2(SO_4)_3$ (20 min) | EtOH | 55.63 | 23.63 | 32.02 |
| 32 | CMC 9H4F | 5.4 | $Al_2(SO_4)_3$1.83%, $B(OH)_3$ 0.9% | 0.1 g BA and 0.14g $Al_2(SO_4)_3$ (20 min) | EtOH | 51.91 | 28.73 | 30.71 |
| 33 | CMC 9H4F | 5.4 | $Al_2(SO_4)_3$ 1.83 %, $B(OH)_3$ 0.9% | 0.1 g BA and 0.14g $Al_2(SO_4)_3$ (20 min) | EtOH | 56.4 | 30.97 | 31.86 |
| 34 | CMC 9H4F | 5.4 | $Al_2(SO_4)_3$ 1.83 %, $B(OH)_3$ 0.9% | 0.1 g BA and 0.14g $Al_2(SO_4)_3$ (20 min) | EtOH | 58.8 | 32.59 | 40.62 |
| 35 | CMC 9H4F | 5.4 | $Al_2(SO_4)_3$ 1.83 %, $B(OH)_3$ 0.9% | 0.1 g BA and 0.15g $Al_2(SO_4)_3$ (20 min) | EtOH | 59 | 37.35 | 39.44 |
| 36 | CMC 9H4F | 5.4 | $Al_2(SO_4)_3$ 1.83%. $B(OH)_3$ 0.9% | 0.1 g BA and 0.15g $Al_2(SO_4)_3$ (20 min) | EtOH | 54.15 | 26.14 | 28.13 |
| 37 | CMC 9H4F | 5.4 | $Al_2(SO_4)_3$1.83%, $B(OH)_3$ 0.9% | 0.15g $Al_2(SO_4)_3$ (20 min) | EtOH | 62.15 | 39.24 | 35.97 |
| 38 | CMC 9H4F | 5.4 | $Al_2(SO_4)_3$ 2.75% | 0.15g $Al_2(SO_4)_3$ (20 min) | EtOH | 56.35 | 32.8 | 29.35 |
| 39 | CMC 9H4F | 5.4 | $Al_2(SO_4)_3$ 1.83%, $B(OH)_3$ 0.9% | 0.1 g BA and 0.125g $Al_2(SO_4)_3$ | EtOH | 48.36 | 25.87 | |
| 40 | CMC 9H4F | 5.4 | $Al_2(SO_4)_3$ 2.75% | 0.14g $Al_2(SO_4)_3$ | EtOH | 52.9 | 30.49 | |
| 41 | CMC 9H4F | 5.4 | $Al_2(SO_4)_3$ 1,85% | 0.14g $Al_2(SO_4)_3$ | EtOH | 51.63 | 23.64 | |
| 42 | CMC 9H4F | 5.4 | $Al_2(SO_4)_3$ 1.83%, $B(OH)_3$ 0.9% | 0.14g $Al_2(SO_4)_3$ wo washing | EtOH | 55.41 | 21.93 | 28.81 |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 43 | CMC 9H4F | 5.4 | $Al_2(SO_4)_3$ 1.38%, B$(OH)_3$ 0.9% | 0.14g $Al_2(SO_4)_3$ wo washing | EtOH | 59.9 | 24.81 | 29.36 |
| 44 | CMC 9H4F | 5.4 | $Al_2(SO_4)_3$ 0.9%, B$(OH)_3$ 0.9% | 0.14g $Al_2(SO_4)_3$ wo washing | EtOH | 56.16 | 21.56 | 30.63 |
| 45 | CMC 9H4F | 5.4 | $Al_2(SO_4)_3$ 2.75% | 0.14g $Al_2(SO_4)_3$ wo washing | EtOH | 57.02 | 25.59 | 31.33 |
| 46 | CMC 9H4F | 5.4 | $Al_2(SO_4)_3$ 1.83%, B$(OH)_3$ 0.9% | 0.15g $Al_2(SO_4)_3$ wo washing | EtOH | 51.31 | 31.24 | |
| 47 | CMC 9H4F | 5.4 | $Al_2(SO_4)_3$ 1.83%, B$(OH)_3$ 0.9% | 0.15g $Al_2(SO_4)_3$ wo washing | EtOH | 52.74 | 22.05 | |
| 48 | CMC 9H4F | 5.4 | $Al_2(SO_4)_3$ 1.83%, B$(OH)_3$ 0.9% | 0.15g $Al_2(SO_4)_3$ wo washing | EtOH | 54.94 | 31.98 | |
| 49 | CMC 9H4F | 5.4 | $Al_2(SO_4)_3$ 1.83%, B$(OH)_3$ 0.9% | 0.15g $Al_2(SO_4)_3$ wo washing | EtOH | 54.12 | 23.07 | |
| 50 | CMC 9H4F | 5.4 | $Al_2(SO_4)_3$ 1.83%, B$(OH)_3$ 0.9% | 0.16g $Al_2(SO_4)_3$ wo washing | EtOH | 56.99 | 38.26 | |
| 51 | CMC 9H4F | 5.4 | $Al_2(SO_4)_3$ 1.83%, B$(OH)_3$ 0.9% | 0.16g $Al_2(SO_4)_3$ wo washing | EtOH | 52.69 | 22.99 | |
| 52 | CMC 9H4F | 5.4 | $Al_2(SO_4)_3$ 1.83%, B$(OH)_3$ 0.9% | 0.16g $Al_2(SO_4)_3$ wo washing | EtOH | 55.3 | 27.09 | |
| 53 | CMC 9H4F | 5.4 | $Al_2(SO_4)_3$ 1.83%, B$(OH)_3$ 0.9% | 0.16g $Al_2(SO_4)_3$ wo washing | EtOH | 53.3 | 20.99 | |
| 54 | CMC 9H4F | 5.4 | $Al_2(SO_4)_3$ 1.83%, B$(OH)_3$ 0.9% | 0.125g $Al_2(SO_4)_3$ wo washing | EtOH | 56.72 | 36.19 | |
| 55 | CMC 9H4F | 5.4 | $Al_2(SO_4)_3$ 1.83%, B$(OH)_3$ 0.9% | 0.14g $Al_2(SO_4)_3$ wo washing | EtOH | 55.21 | 26.92 | |
| 56 | CMC 9H4F | 5.4 | $Al_2(SO_4)_3$ 1.83%, B$(OH)_3$ 0.9% | 0.15g $Al_2(SO_4)_3$ wo washing | EtOH | 52.03 | 22.84 | |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 57 | CMC 9H4F | 5.4 | $Al_2(SO_4)_3$ 1.83%, B $(OH)_3$ 0.9% | 0.16g $Al_2$ $(SO_4)_3$ wo washing | EtOH | 50.54 | 22.35 | |
| 58 | CMC 9H4F | 5.4 | $Al_2(SO_4)_3$ 1.83%, B $(OH)_3$ 0.9% | 0.17g $Al_2$ $(SO_4)_3$ wo washing | EtOH | 50.51 | 21.87 | |
| 59 | CMC 9H4F | 5.4 | $Al_2(SO_4)_3$ 1.83%, B $(OH)_3$ 0.9% | 0.18g $Al_2$ $(SO_4)_3$ wo washing | EtOH | 48.95 | 21.16 | |
| 60 | CMC 9H4F | 5.4 | $Al_2(SO_4)_3$ 1.83%, B $(OH)_3$ 0.9% | 0.18g $Al_2$ $(SO_4)_3$ wo washing | EtOH | 48.22 | 19.81 | |
| 61 | CMC 9H4F | 5.5 | $Al_2(SO_4)_3$ 2.75% | 0.16g $Al_2$ $(SO_4)_3$ wo washing | EtOH | 48.54 | 14.29 | |
| 62 | CMC 9H4F | 5.5 | $Al_2(SO_4)_3$ 2.75% | 0.16g $Al_2$ $(SO_4)_3$ wo washing | EtOH | 57.53 | 27 | |
| 63 | CMC 9H4F | 5.4 | $Al_2(SO_4)_3$ 3.63% | 0.14g $Al_2$ $(SO_4)_3$ wo washing | EtOH | 65.09 | 33.7 | |
| 64 | CMC 9H4F | 5.4 | $Al_2(SO_4)_3$ 4.5% | 0.14g $Al_2$ $(SO_4)_3$ wo washing | EtOH | 66.19 | 38.01 | |
| 65 | CMC 9H4F | 5.5 | $Al_2(SO_4)_3$ 2.75% | 0.16g $Al_2$ $(SO_4)_3$ wo washing | i-PrOH | 58.59 | 29.87 | |
| 66 | CMC 9H4F | 5.5 | $Al_2(SO_4)_3$ 2.75% | 0.16g $Al_2$ $(SO_4)_3$ wo washing | i-PrOH | 53.88 | 26.15 | |
| 67 | CMC 9H4F | 5.5 | $Al_2(SO_4)_3$ 1.83%, B $(OH)_3$ 0.9% | 0.16g $Al_2$ $(SO_4)_3$ wo washing | i-PrOH | 67.09 | 33.28 | 29.02 |
| 68 | CMC 9H4F | 5.5 | $Al_2(SO_4)_3$ 1.83%, B $(OH)_3$ 0.9% | 0.16g $Al_2$ $(SO_4)_3$ wo washing | i-PrOH | 71.19 | 29.36 | 28.47 |
| 69 | CMC 9H4F | 5.5 | $Al_2(SO_4)_3$ 1.85% | 0.17g $Al_2$ $(SO_4)_3$ wo washing | i-PrOH | 63.53 | 28.58 | 22.15 |
| 70 | CMC 9H4F | 5.5 | $Al_2(SO_4)_3$ 1.85% | 0.17g $Al_2$ $(SO_4)_3$ wo washing | i-PrOH | 55.18 | 20.25 | 22.24 |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 71 | CMC 9H4F | 5.5 | $Al_2(SO_4)_3$ 1.84%. B(OH)$_3$ 0.46% | 0. 1 3g $Al_2(SO_4)_3$ wo washing | i-PrOH | 36.78 | 7.1 | |
| 72 | CMC 9H4F | 5.5 | $Al_2(SO_4)_3$ 1.83 %, B(OH)$_3$ 0.9% | 0.16g $Al_2(SO_4)_3$ wo washing | i-PrOH | 57.89 | 18.42 | 23.91 |
| 73 | CMC 9H4F | 5.5 | $Al_2(SO_4)_3$ 1.83%, B(OH)$_3$ 0.9% | 0.16g $Al_2(SO_4)_3$ wo washing | i-PrOH | 52.98 | 12.91 | 23.62 |
| 74 | CMC 9H4F | 5.5 | $Al_2(SO_4)_3$ 1.85% | 0.17g $Al_2(SO_4)_3$ wo washing | i-PrOH | 44.74 | 12.08 | 19.42 |
| 75 | CMC 9H4F | 5.5 | $Al_2(SO_4)_3$ 1.85% | 0.17g $Al_2(SO_4)_3$ wo washing | i-PrOH | 49.53 | 15.92 | 25.2 |
| 76 | PA-CMC | 5.4 | $Al_2(SO_4)_3$ 0.23 %, B(OH)$_3$ 0.9% | 0.1g BA and 0.14g $Al_2(SO_4)_3$ | EtOH | 48.11 | 22 | 29.7 |
| 77 | PA-CMC | 5.4 | $Al_2(SO_4)_3$ 0.69%, B(OH)$_3$ 0.9% | 0.1g BA and 0.14g $Al_2(SO_4)_3$ | EtOH | 46.89 | 20.14 | 25.48 |
| 78 | PA-CMC | 5.4 | $Al_2(SO_4)_3$ 2.72 %, B(OH)$_3$ 0.9% | 0.05g BA and 0.16g $Al_2(SO_4)_3$ | EtOH | 50.92 | 29.78 | |
| 79 | PA-CMC | 5.4 | $Al_2(SO_4)_3$ 1.83%, B(OH)$_3$ 0.9% | 0.05g BA and 0.16g $Al_2(SO_4)_3$ | EtOH | 49.28 | 24.86 | |
| 80 | PA-CMC | 5.4 | $Al_2(SO_4)_3$ 0.9%, B(OH)$_3$ 0.9% | 0.05g BA and 0.16g $Al_2(SO_4)_3$ | EtOH | 51.46 | 33.67 | |
| 81 | PA-CMC | 5.4 | $Al_2(SO_4)_3$ 2.75% | 0.18g $Al_2(SO_4)_3$ w wash | EtOH | 44.78 | 24.99 | |
| 82 | PA-CMC | 5.4 | $Al_2(SO_4)_3$ 2.72 %, B(OH)$_3$ 0.9% | 0.18g $Al_2(SO_4)_3$ w wash | EtOH | 51.11 | 26.49 | |
| 83 | PA-CMC | 5.4 | $Al_2(SO_4)_3$ 1.83%, B(OH)$_3$ 0.9% | 0.18g $Al_2(SO_4)_3$ w wash | EtOH | 52.74 | 33.59 | |
| 84 | PA-CMC | 5.4 | $Al_2(SO_4)_3$ 0.9%, B(OH)$_3$ 0.9% | 0.18g $Al_2(SO_4)_3$ w wash | EtOH | 51.25 | 32.2 | |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 85 | PA-CMC | 5.4 | $Al_2(SO_4)_3$ 2.75% | 0.18g $Al_2(SO_4)_3$ w wash | EtOH | 45.64 | 24.85 | |
| 86 | PA-CMC | 5.4 | $Al_2(SO_4)_3$ 2.72 %, B(OH)$_3$ 0.9% | 0.18g $Al_2(SO_4)_3$ w wash | EtOH | 52.09 | 26.85 | |
| 87 | PA-CMC | 5.4 | $Al_2(SO_4)_3$ 1.83%, B(OH)$_3$ 0.9% | 0.18g $Al_2(SO_4)_3$ w wash | EtOH | 49.5 | 25.89 | |
| 88 | PA-CMC | 5.4 | $Al_2(SO_4)_3$ 0.9%, B(OH)$_3$ 0.9% | 0.20g $Al_2(SO_4)_3$ w wash | EtOH | 50.09 | 28.49 | |
| 89 | PA-CMC | 5.4 | $Al_2(SO_4)_3$ 2.75% | 0.18g $Al_2(SO_4)_3$ w wash | EtOH | 45.47 | 23.07 | |
| 90 | PA-CMC | 5.4 | $Al_2(SO_4)_3$ 2.72 %, B(OH)$_3$ 0.9% | 0. 18g $Al_2(SO_4)_3$ w wash | EtOH | 44.04 | 18.61 | |
| 91 | PA-CMC | 5.4 | $Al_2(SO_4)_3$ 1.83%, B(OH)$_3$ 0.9% | 0.18g $Al_2(SO_4)_3$ w wash | EtOH | 48.37 | 23.07 | |
| 92 | PA-CMC | 5.4 | $Al_2(SO_4)_3$ 0.9%, B(OH)$_3$ 0.9% | 0.20g $Al_2(SO_4)_3$ w wash | EtOH | 46.14 | 20.36 | |
| 93 | PA-CMC | 5.4 | $Al_2(SO_4)_3$ 2.75% | 0.18g $Al_2(SO_4)_3$ w wash | EtOH | 47.23 | 19.85 | |
| 94 | PA-CMC | 5.4 | $Al_2(SO_4)_3$ 0.23 %, B(OH)$_3$ 0.9% | 0.20g $Al_2(SO_4)_3$ w wash | EtOH | 49.23 | 26.34 | |
| 95 | PA-CMC | 5.4 | $Al_2(SO_4)_3$ 0.46%, B(OH)$_3$ 0.9% | 0.20g $Al_2(SO_4)_3$ w wash | EtOH | 45.65 | 20.12 | |
| 96 | PA-CMC | 5.4 | $Al_2(SO_4)_3$ 0.92%, B(OH)$_3$ 0.92% | 0.20g $Al_2(SO_4)_3$ w wash | EtOH | 43.59 | 14.04 | |
| 97 | PA-CMC | 5.4 | $Al_2(SO_4)_3$ 0.93% | 0.20g $Al_2(SO_4)_3$ w wash | EtOH | 44.33 | 21.46 | |
| 98 | PA-CMC | 5.4 | $Al_2(SO_4)_3$ 2.72 %, B(OH)$_3$ 0.9% | 0.18g $Al_2(SO_4)_3$ w wash | EtOH | 49.3 | 23.37 | |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 99 | PA-CMC | 5.4 | $Al_2(SO_4)_3$ 1.83%, B$(OH)_3$ 0.9% | 0.18g $Al_2$ $(SO_4)_3$ w wash | EtOH | 51.89 | 26.54 | |
| 100 | PA-CMC | 5.4 | $Al_2(SO_4)_3$ 0.9%, B$(OH)_3$ 0.9% | 0.19g $Al_2$ $(SO_4)_3$ w wash | EtOH | 55.17 | 30.5 | |
| 101 | PA-CMC | 5.4 | $Al_2(SO_4)_3$ 2.75% | 0.18g $Al_2$ $(SO_4)_3$ w wash | EtOH | 53.54 | 23.73 | |
| 102 | PA-CMC | 5.4 | $Al_2(SO_4)_3$ 2.72 %, B$(OH)_3$ 0.9% | 0.18g $Al_2$ $(SO_4)_3$ w wash | EtOH | 53.61 | 26.9 | |
| 103 | PA-CMC | 5.4 | $Al_2(SO_4)_3$ 1.83%, B$(OH)_3$ 0.9% | 0.18g $Al_2$ $(SO_4)_3$ w wash | EtOH | 52.88 | 28.25 | |
| 104 | PA-CMC | 5.4 | $Al_2(SO_4)_3$ 0.9%, B$(OH)_3$ 0.9% | 0.20g $Al_2$ $(SO_4)_3$ w wash | EtOH | 53.24 | 29.94 | |
| 105 | PA-CMC | 5.4 | $Ab(SO_4)_3$ 2.75% | 0.18g $Al_2$ $(SO_4)_3$ w wash | EtOH | 51.06 | 25.43 | |
| 106 | PA-CMC | 5.5 | $Al_2(SO_4)_3$ 0.9%, B$(OH)_3$ 0.9% | 0.19g $Al_2$ $(SO_4)_3$ w wash | EtOH | 50.6 | 20.03 | |
| 107 | PA-CMC | 5.6 | $Al_2(SO_4)_3$ 0.9% | 0.22g $Al_2$ $(SO_4)_3$ w wash | EtOH | 46.67 | 17.3 | |
| 108 | PA-CMC | None | $Al_2$ $(SO_4)_3$0.98%. B$(OH)_3$ 0.98% | 0.19g $Al_2$ $(SO_4)_3$ w wash | EtOH | 57.07 | 28.68 | |
| 109 | PA-CMC | None | $Al_2(SO_4)_3$ 0.99% | 0.22g $Al_2$ $(SO_4)_3$ w wash | EtOH | 51.77 | 27.17 | |
| 110 | LV-PN | 5.4 | $Al_2(SO_4)_3$ 3.63% | 0.17g $Al_2$ $(SO_4)_3$ w wash | EtOH | 54.11 | 20.57 | |
| 111 | LV-HW | 5.4 | $Al_2$ $(SO_4)_3$3.63% | 0.17g $Al_2$ $(SO_4)_3$ w wash | EtOH | 57.05 | 23.92 | |
| 112 | LV-FIR | 5.4 | $Al_2(SO_4)_3$ 3.63% | 0.17g $Al_2$ $(SO_4)_3$ w wash | EtOH | 59.68 | 24.7 | |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 113 | KL-SW | 5.4 | $Al_2(SO_4)_3$ 3.63% | 0.17g $Al_2(SO_4)_3$ w wash | EtOH | 58.06 | 21.12 | |
| 114 | LV-PN | 5.6 | $Al_2(SO_4)_3$ 0.46% | 0.16g $Al_2(SO_4)_3$ w wash | EtOH | 59.85 | 29.3 | 36.6 |
| 115 | LV-PN | 5.6 | $Al_2(SO_4)_3$ 0.9% | 0.24g $Al_2(SO_4)_3$ w wash | EtOH | 51.57 | 21.85 | 33.53 |
| 116 | LV-PN | 5.6 | $Al_2(SO_4)_3$ 1.85% | 0.22g $Al_2(SO_4)_3$ w wash | EtOH | 55.28 | 27.28 | 33.06 |
| 117 | LV-PN | 5.4 | $Al_2(SO_4)_3$ 3.63% | 0.20g $Al_2(SO_4)_3$ w wash | EtOH | 54.19 | 31.26 | 26.73 |
| 118 | LV-PN | 5.5 | $Al_2(SO_4)_3$ 1.85% | 0.14g $Al_2(SO_4)_3$ w wash | EtOH | 44.85 | 27.8 | 19.1 |
| 119 | LV-HW | 5.5 | $Al_2(SO_4)_3$ 1.85% | 0. 1 4g $Al_2(SO_4)_3$ w wash | EtOH | 47.76 | 29.45 | 14.16 |
| 120 | LV-FIR | 5.5 | $Al_2(SO_4)_3$ 1.85% | 0.14g $Al_2(SO_4)_3$ w wash | EtOH | 46.58 | 31.94 | 32.56 |
| 121 | KL-SW | 5.5 | $Al_2(SO_4)_3$ 1.85% | 0.14g $Al_2(SO_4)_3$ w wash | EtOH | 49.31 | 28.05 | 28.43 |
| 122 | LV-PN | 5.4 | $Al_2(SO_4)_3$ 3.63% | 0.16g $Al_2(SO_4)_3$ w wash | EtOH | 55.88 | 22.17 | 22 |
| 123 | LV-HW | 5.4 | $Al_2(SO_4)_3$ 3.63% | 0. 1 6g $Al_2(SO_4)_3$ w wash | EtOH | 53.75 | 21.49 | 21.44 |
| 124 | LV-FIR | 5.4 | $Al_2(SO_4)_3$ 3.63% | 0. 1 6g $Al_2(SO_4)_3$ w wash | EtOH | 51.77 | 20.39 | 22.48 |
| 125 | KL-SW | 5.4 | $Al_2(SO_4)_3$ 3.63% | 0.16g $Al_2(SO_4)_3$ w wash | EtOH | 51.82 | 19.55 | 23.24 |
| 126 | LV-PN | 5.5 | $Al_2(SO_4)_3$ 1.85% | 0.14g $Al_2(SO_4)_3$ w wash | EtOH | 46.5 | 26.69 | |

(continued)

| Sample | CMC | Guar Gum (% wgt total wgt) | First crosslinking agent (% wgt total wgt, applied) | Second crosslinking agent/2g | Fiber forming solvent | Free Swell (g/g) | CRC (g/g) | AUL (g/g) |
|---|---|---|---|---|---|---|---|---|
| 127 | LV-HW | 5.5 | $Al_2(SO_4)_3$ 1.85% | 0.14g $Al_2(SO_4)_3$ w wash | EtOH | 48.29 | 29.32 | |
| 128 | LV-FIR | 5.5 | $Al_2(SO_4)_3$ 1.85% | 0. 1 4g $Al_2(SO_4)_3$ w wash | EtOH | 53.13 | 30.91 | |
| 129 | KL-SW | 5.5 | $Al_2(SO_4)_3$ 1.85% | 0.14g $Al_2(SO_4)_3$ w wash | EtOH | 50.05 | 29.59 | |

Table 2. Metal Analysis Data for Selected Superabsorbent Fiber

| Sample | CMC | Guar Gum (% wgt total wgt) | First crosslinking agent (% wgt total wgt, applied) | Second crosslinking agent/2g | Fiberforming solvent | Al (mg/kg) | B (mg/kg) |
|---|---|---|---|---|---|---|---|
| 1A | CMC 9H4F | 5.4 | $Al_2(SO_4)_3$ 2.72 $B(OH)_3$ 0.9% | None | EtOH | 3865 | <60 |
| 1 | CMC 9H4F | 5.4 | $Al_2(SO_4)_3$ 2.72 %, B $(OH)_3$ 0.9% | 0. 1 g BA and 0. 1 25g $Al_2(SO_4)_3$ | EtOH | 9785 | 260 |
| 2A | CMC 9H4F | 5.4 | $Al_2(SO_4)_3$ 1.83%, B $(OH)_3$ 0.9% | None | EtOH | 2555 | <60 |
| 2 | CMC 9H4F | 5.4 | $Al_2(SO_4)_3$ 1.83%, | 0.1g BA and 0.125g $Al_2(SO_4)_3$ | EtOH | 9465 | 205 |
| 3A | CMC 9H4F | 5.4 | $Al_2(SO_4)_3$ 0.9%, $B(OH)_3$ 0.9% | None | EtOH | 1210 | <60 |
| 3 | CMC 9H4F | 5.4 | $Al_2(SO_4)_3$ 0.9%, $B(OH)_3$ 0.9% | 0.1g BA and 0.125g $Al_2(SO_4)_3$ | EtOH | 7920 | 160 |
| 4A | CMC 9H4F | 5.4 | $Al_2(SO_4)_3$ 2.75%. | None | EtOH | 3890 | <60 |
| 4 | CMC 9H4F | 5.4 | $Al_2(SO_4)_3$ 2.75%. | 0.1g BA and 0.125g $Al_2(SO_4)_3$ | EtOH | 10750 | 195 |

Example 9

A solution of CMC 9H4F 10.0 g OD in 450 ml deionized (DI) water was prepared with vigorous stirring to obtain a CMC solution. Guar gum (0.6 g) was dissolved in 25 ml DI water and mix well with the CMC solution. The solution was stirred for one hour to allow complete mixing of the two polymers.

The polymer mixture was blended in the blender. Fully dissolve basic dihydroxy aluminum acetate stabilized with boric acid (purchased from Sigma-Aldrich Fine Chemicals) 0.125g in 25 ml DI water. Transfer the aluminum acetate stabilized with boric acid solution to the polymer solution and blend for five minutes to mix to provide a gel. Leave the gel at ambient temperature (25°C) for one hour.

Example 10

A solution of CMC 9H4F 10.0 g OD in 950 ml deionized (DI) water was prepared with vigorous stirring to obtain a CMC solution. Guar gum (0.6 g) was dissolved in 25 ml DI water and mix well with the CMC solution. The solution was stirred for one hour to allow complete mixing of the two polymers.

The polymer mixture was blended in the blender. Fully dissolve basic dihydroxy aluminum acetate stabilized with boric acid (purchased from Sigma-Aldrich Fine Chemicals) 0.125g in 25 ml DI water. Transfer the aluminum acetate stabilized with boric acid solution to the polymer solution and blend for five minutes to provide a gel. Leave the gel at ambient temperature (25°C) for one hour.

Example 11

The aqueous gels described above in Examples 9 and 10 were extruded in a wet spinning extruder to form a gel fiber. The gel from the Example 9 was a 2% by weight solution and the gel from Example 10 a 1 % by weight solution. The gel fiber was placed in a denatured ethanol solvent to precipitate the fibers. There was no second crosslinking. The filaments formed were 700 $\mu$m in diameter. The following table gives the amount of gel in solution and the free swell, centrifuge capacity and AUL of the fibers. Free swell, centrifuge capacity and AUL are in grams absorbed per gram of fiber.

Table 3

| Example | gel in solution, % | Free swell | centrifuge capacity | AUL |
|---------|--------------------|------------|---------------------|-----|
| 9 | 2 | 31.2 | 13.32 | 18.71 |
| 9 | 2 | 27.16 | 14.03 | 22.95 |
| 10 | 1 | 34.51 | 17.07 | 13.23 |

Example 12

In this example, the preparation of representative mixed polymer composite fibers crosslinked with fresh aluminum sulfate and fresh aluminum sulfate is described. A solution of Weyerhaeuser pine pulp CMC (40g OD) in 900 ml deionized water was prepared with vigorous stirring to obtain a CMC solution. Guar gum (2.4 g) was dissolved in 50 ml DI water and mixed with the CMC solution. The solution was stirred for one hour to allow complete mixing of the two polymers.

Weigh 0.8g of fresh aluminum sulfate octadecahydrate and dissolve in 50 ml DI water. Transfer aluminum sulfate solution to the polymer solution and blend for 5 minutes to mix well. Leave the gel at ambient temperature (25°C) for one hour.

The gel was formed into fibers using wet spinning (one orifice with hole diameter of 500 micron).

The gel fibers entered a water-miscible solvent bath containing 1200 ml water and 3600 ml isopropanol containing a second crosslinker. The crosslinker concentration in the following table is based on the amount of crosslinker per 4g dry gel extruded. The second crosslinker was also fresh aluminum sulfate. Each portion of precipitated fiber was then soaked in 500 ml of isopropanol and mixed for 10 minutes. The fibers were then dried.

The following table gives the speed of the gel through the orifice, the amount of crosslinker in the bath, and the free swell, and centrifuge capacity of the composite fiber. Free swell, centrifuge capacity and AUL are in grams absorbed per gram of fiber.

Table 4

| gel rate. g/min | crosslinker % | free swell | centrifuge capacity |
|-----------------|---------------|------------|---------------------|
| 30 | 0.05 | 35.4 | 18.54 |
| 30 | 0.072 | 41.09 | 25.16 |

(continued)

| gel rate. g/min | crosslinker % | free swell | centrifuge capacity |
|---|---|---|---|
| 30 | 0.084 | 40.83 | 26.71 |
| 30 | 0.148 | 29.3 | 13.69 |
| 10 | 0.2 | 38.79 | 21.15 |

While illustrative embodiments have been illustrated and described, it will be appreciated that various changes can be made therein without departing from the spirit and scope of the invention.

The embodiments of the invention in which an exclusive property or privilege is claimed are defined as follows:

**Claims**

1.  A mixed polymer composite fiber, comprising a carboxyalkyl cellulose and a galactomannan polymer or a glucomannan polymer and further comprising a plurality of intra-fiber multivalent metal crosslinks wherein the metal ions are selected from the group consisting of aluminum, boron, bismuth, titanium, and zirconium ions, and mixtures thereof.

2.  The fiber of Claim 1, wherein the carboxyalkyl cellulose is present in an amount from about 60 to about 99 percent by weight based on the total weight of the fiber.

3.  The fiber of Claim 1, wherein the galactomannan polymer or glucomannan polymer is present in an amount from about 1 to about 20 percent by weight based on the total weight of the fiber.

4.  A method for making the mixed polymer composite fibers of claims 1-3, comprising:

    (a) blending a carboxyalkyl cellulose and a galactomannan polymer or glucomannan polymer in water to provide an aqueous solution;
    (b) treating the aqueous solution with a first crosslinking agent to provide a gel;
    (c) mixing the gel with a water-miscible solvent and stirring the gel-solvent mixture to provide fibers; and
    (d) treating the fibers with a second crosslinking agent to provide crosslinked mixed polymer composite fibers

    wherein the first and second crosslinking agents are selected from the group consisting of aluminum compounds, titanium compounds, bismuth compounds, boron compounds, and zirconium compounds.

5.  A method for making the mixed polymer composite fibers of any one of claims 1 to 3 comprising:

    (a) blending a carboxyalkyl cellulose and a galactomannan polymer or glucomannan polymer in water to provide an aqueous solution;
    (b) treating the aqueous solution with a first crosslinking agent to provide a gel; wherein the first crosslinking agent is selected from the group consisting of aluminum compounds, titanium compounds, bismuth compounds, boron compounds, and zirconium compounds,
    (c) forming gel fibers from the gel using melt blowing, centrifugal spinning, wet spinning, or dry-jet wet spinning,
    (d) treating the gel fibers with a water-miscible solvent to provide mixed polymer composite fibers.

6.  The method of Claim 5 further comprising treating the gel fibers with a second crosslinking agent during step (d), wherein the second crosslinking agent is selected from the group consisting of aluminum compounds, titanium compounds, bismuth compounds, boron compounds, and zirconium compounds.

7.  The method of any of Claims 4 through 6, wherein the crosslinking agent is applied in an amount from about 0.1 to about 20 percent by weight based on the total weight of crosslinked fibers.

8.  The method of any of Claims 4 through 7, wherein the aqueous solution comprises from about 60 to about 99 percent by weight carboxyalkyl cellulose based on the total weight of mixed polymer composite fibers.

9. The method of any of Claims 4 through 8, wherein the aqueous solution comprises from about 1 to about 20 percent by weight galactomannan or glucomannan polymer based on the total weight of mixed polymer composite fibers.

100μm

FIG. 1.

10µm

**FIG. 2.**

20µm

FIG. 3.

Fig. 4

Fig. 5

32 ~

36

38 ~ 38

34 ~

40 ~

42 ~ 44 ~

*Fig. 6*

55 53 54 55

51 51

50 ~ 50 ~

52 ~ 36 52 ~

40

*Fig 7*

Fig. 8

**European Patent Office**

**EUROPEAN SEARCH REPORT**

Application Number

EP 07 25 3765

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 4 128 692 A (REID ALBERT R) 5 December 1978 (1978-12-05) * claims 1-17 * * column 2, lines 1-30 * | 1-3 | INV. A61L15/28 |
| X | US 5 847 031 A (KLIMMEK HELMUT [DE] ET AL) 8 December 1998 (1998-12-08) * claim 1 * | 1-3 | |
| X | US 4 143 163 A (HUTCHISON BRUCE R ET AL) 6 March 1979 (1979-03-06) * column 4, lines 45-60 * | 1-3 | |

TECHNICAL FIELDS SEARCHED (IPC)

A61L

The present search report has been drawn up for all claims

| Place of search | Date of completion of the search | Examiner |
|---|---|---|
| Munich | 13 March 2008 | Cismaru, L |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 07 25 3765

This annex lists the patent family members relating to the patent documents cited in the above-mentioned European search report.
The members are as contained in the European Patent Office EDP file on
The European Patent Office is in no way liable for these particulars which are merely given for the purpose of information.

13-03-2008

| Patent document cited in search report | Publication date | Patent family member(s) | Publication date |
|---|---|---|---|
| US 4128692 A | 05-12-1978 | NONE | |
| US 5847031 A | 08-12-1998 | AU 681076 B2 | 21-08-1997 |
| | | AU 4029593 A | 21-11-1994 |
| | | BG 62856 B1 | 29-09-2000 |
| | | BG 100174 A | 31-01-1997 |
| | | DE 59307094 D1 | 11-09-1997 |
| | | WO 9425520 A1 | 10-11-1994 |
| | | EP 0700415 A1 | 13-03-1996 |
| | | ES 2107022 T3 | 16-11-1997 |
| | | FI 955228 A | 01-11-1995 |
| | | HU 75499 A2 | 28-05-1997 |
| | | JP 8510487 T | 05-11-1996 |
| | | JP 4032261 B2 | 16-01-2008 |
| | | SK 131495 A3 | 08-01-1997 |
| US 4143163 A | 06-03-1979 | NONE | |

EPO FORM P0459

For more details about this annex : see Official Journal of the European Patent Office, No. 12/82